# EUROPEAN PATENT APPLICATION

(11) **EP 3 715 847 A1**
(43) Date of publication of application: **30.09.2020**
(21) Application number: 20158626.0
(22) Date of filing: 20.02.2020
(51) Int. Cl.: G01N 33/569, C07K 14/005, C07K 14/165

(54) **A METHOD AND REAGENTS FOR THE DIAGNOSIS OF SARS-COV-2**

(71) Applicant: Euroimmun Medizinische Labordiagnostika AG, 23560 Lübeck (DE); Charité - Universitätsmedizin Berlin, 10117 Berlin (DE)
(72) Inventor: The designation of the inventor has not yet been filed

(57) **Abstract**

The present invention relates to a method for diagnosing a SARS-CoV-2 infection comprising the step of detecting the presence or absence of an IgA class antibody to SEQ ID NO1 in a sample from a subject, a method for the differential diagnosis of a coronavirus infection, a use of an IgA class antibody to SEQ ID NO1 for diagnosing a SARS-CoV-2 infection or for the differential diagnosis of a coronavirus infection, preferably for distinguishing between a SARS-CoV-2, MERS and NL63, 229E, OC43 and HKU1 infection, and a kit comprising a polypeptide comprising SEQ ID NO1 or a variant thereof, preferably coated to a diagnostically useful carrier and one or more, preferably all reagents from the group comprising an antibody to SEQ ID NO1, a washing buffer, a means for detecting the presence of an IgA class antibody, preferably a secondary antibody binding specifically to IgA class antibodies, preferably comprising a detectable label, and a dilution buffer.

## Description

The present invention relates to a method for diagnosing a SARS-CoV-2 infection comprising the step of detecting the presence or absence of an antibody to SEQ ID NO1, preferably preferably IgA class antibody, in a sample from a subject, a method for the differential diagnosis of a coronavirus infection, a use of an antibody to SEQ ID NO1, preferably IgA class antibody for diagnosing a SARS-CoV-2 infection or for the differential diagnosis of a coronavirus infection, preferably for distinguishing between a SARS-CoV-2, MERS and NL63, 229E, OC43 and HKU1 infection, and a kit comprising a polypeptide comprising SEQ ID NO1 or a variant thereof, preferably coated to a diagnostically useful carrier and one or more, preferably all reagents from the group comprising an antibody to SEQ ID NO1, a washing buffer, a means for detecting the presence of an antibody, preferably IgA class antibody, preferably a secondary antibody binding specifically to IgA class antibodies, preferably comprising a detectable label, and a dilution buffer.

At the end of 2019, a rising number of pneumonia patients with unknown pathogen emerged from Wuhan, the capital of Hubei province, China, to nearly the entirety of China. A novel coronavirus was isolated and based on its phylogeny, taxonomy and established practice, the Coronavirus Study Group (CSG) recognized it as a sister to severe acute respiratory syndrome coronaviruses (SARS-CoVs) and labeled it as severe acute respiratory syndrome coronavirus 2 (SARS-CoV-2).

Although SARS-CoV-2 is generally less pathogenic than SARS-CoV and Middle East respiratory syndrome coronavirus (MERS-CoV), it has a relatively high transmissibility. Since symptoms may be mild and may be confused with a cold, there is the danger that patients are unaware that they have been infected and may help the virus spread further.

Corman *et al.* published a real time RT-PCR based assay for the detection of SARS-CoV-2 (Corman et al. (2020) Diagnostic detection of 2019-nCoV by real-time RT-PCR, https://www.who.int/docs/default-source/coronaviruse/protocol-v2-1.pdf?sfvrsn=a9ef618c 2).

WO14045254 discloses assays for diagnosing a MERS infection.

Hsueh *et al.,* reported that IgG could be detected as early as four days after the onset of a SARS infection, simultaneously or one day earlier than IgM and IgA (Hsue, P. R., Huang, L. M., Chen, P. J., Kao, C. L., and Yang P. C. (2004) Chronological evolution of IgM, IgA, IgG and neutralization antibodies after infection with SARS-associated coronavirus, Clinical Microbiology and Infection, 10(12), 1062-1066.

However, PCR-based assays have several shortcomings. In particular, a sample from the upper respiratory tract of the patient is required. Improper recovery of such a sample may lead to false-negative results. Moreover, the results may not reflect the disease status of the patient. Therefore, a serological assay would be desirable.

Therefore, a problem underlying the present invention is to provide an assay and reagents for the early serological detection of SARS-CoV-2.

Another problem underlying the present invention is to provide an assay that may be used to distinguish known coronavirus infections.

The problems are solved by the subject matter of the independent and dependent claims.

In a first aspect, the problem is solved by a method for diagnosing a SARS-CoV-2 infection comprising the step of detecting the presence or absence of an to SEQ ID NO1, preferably IgM, IgG or IgA class antibody, more preferably IgA class antibody, in a sample from a subject.

In a second aspect, the problem is solved by a method for the differential diagnosis of a coronavirus infection, preferably for distinguishing between a SARS-CoV; preferably SARS-CoV-2; MERS, NL63, 229E, OC43 and HKU1 infection, comprising the step of detecting the presence or absence of an antibody to SEQ ID NO1 in a sample from a subject, preferably an IgA and/or IgG class antibody, more preferably an IgA class antibody.

In a preferred embodiment, the presence of an IgG and/or IgM class antibody to SEQ ID NO1 is detected in addition to an IgA class antibody to SEQ ID NO1.

In a preferred embodiment, the sample is a blood sample, preferably selected from the group comprising whole blood, serum or plasma.

In a preferred embodiment, the antibody is detected using a labeled secondary antibody, preferably binding to IgA class antibodies.

In a preferred embodiment, the antibody, preferably IgA class antibody, is detected using a method selected from the group comprising colorimetry, immunofluorescence, detection of enzymatic activity, chemiluminscence and radioactivity.

In a preferred embodiment, the infection is detected at an early stage.

In a 3^{rd} aspect, the problem is solved by a use of an antibody to SEQ ID NO1, preferably IgA class antibody, for diagnosing a SARS-CoV-2 infection or for the differential diagnosis of a coronavirus infection, preferably for distinguishing between a SARS-CoV-2, MERS, NL63, 229E, OC43 and HKU1 infection.

In a preferred embodiment, the use is for the early diagnosis of a SARS-CoV-2 infection.

In a 4^{th} aspect, the problem is solved by a kit comprising a polypeptide comprising SEQ ID NO1 or a variant thereof, preferably coated to a diagnostically useful carrier and one or more, preferably all reagents from the group comprising an antibody to SEQ ID NO1, a washing buffer, a means for detecting the presence of an antibody to SEQ ID NO1, preferably an IgA class antibody, preferably a secondary antibody binding specifically to IgA class antibodies, preferably comprising a detectable label, and a dilution buffer.

In a preferred embodiment, the diagnostically useful carrier is selected from the group comprising a bead, preferably a paramagnetic bead, a test strip, a microtiter plate, a membrane, preferably from the group comprising western blot, line blot and dot blot, a lateral flow device, a glass surface, a slide, a microarray and a biochip and is preferably a microtiter plate.

In a preferred embodiment, the kit comprises two or more, preferably three or more calibrators.

In a preferred embodiment, each calibrator is a recombinant antibody binding to SEQ ID NO1 which is preferably recognized by a secondary antibody binding to IgA class antibodies.

In a 6^{th} aspect, the problem is solved by a use of a polypeptide comprising SEQ ID NO1 or a variant thereof or an antibody to SEQ ID NO1, preferably IgA class antibody, for the manufacture of a diagnostic kit.

In a 7^{th} aspect, the problem is solved by a use of a recombinant antibody binding to SEQ ID NO1 which is preferably recognized by a secondary antibody binding to IgA class antibodies as a calibrator for the early diagnosis of a SARS-CoV-2 infection.

The present invention is based on the inventors' surprising finding that antibodies against SEQ ID NO1 can be detected at an early stage of the infection, with IgA class antibodies becoming detectable earlier than IgG antibodies. Also, there is no cross reactivity with a range of antibodies in samples from patients infected with other coronaviruses.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a treatment. In other words, the term "diagnosis" comprises not only diagnosing, but also prognosticating and/or monitoring the course of a disease or disorder. The subject is likely or more likely to suffer from a SARS-CoV-2 infection if an IgA and/or IgG and/or IgM antibody to SEQ ID NO1 is detected in samples from them.

The sample is preferably a mammalian, more preferably a human sample.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. According to the invention, it can be distinguished if a patient, preferably one already suspected to have a coronavirus infection, suffers from a SARS, preferably SARS-CoV-1 and/or SARS-CoV-2 infection, or another coronavirus infection, preferably from the group comprising MERS, NL63, 229E, OC43 and HKU1. In a preferred embodiment, the term "SARS-CoV-2", as used herein, refers to a virus characterized by the genome deposited on GenBank under accession code MN908947 and derivatives thereof having at least 80, preferably 85, preferably 88, preferably 90, preferably 91, preferably 92, preferably 93, preferably 94, preferably 95, preferably 96, preferably 97, preferably 98, preferably 99, preferably 99.5, preferably 99.8, preferably 99.9 or 99.99 percent sequence identity over the entire genome nucleotide sequence. All data base entries used herein correspond to the version online at the earliest priority or filing data of the application.

In a preferred embodiment, the term "diagnosis" means that the method or product or use may be used for aiding in the diagnosis of a disease or identifying a subject a risk of suffering from a disease. The term "diagnosis" may also refer to a method or agent used to choose the most promising treatment regime for a patient. In other words, the method or agent may relate to selecting a treatment regimen for a subject.

In a preferred embodiment, the method according to the present invention comprises the step providing the diagnostically useful carrier and a sample from a patient suspected of being infected, preferably a mammalian, more preferably a human patient. The carrier is coated with the polypeptide comprising SEQ ID NO1 or variant thereof. The carrier may then be contacted with the sample under conditions allowing for binding of any antibodies to the polypeptide comprising SEQ ID NO1 or variant thereof. The sample may then be removed and the carrier with the cell may be washed to remove any remaining sample. A secondary antibody or similar reagent or means binding to the antibody and carrying a detectable label may then be contacted with the carrier under conditions allowing formation of a complex between any bound antibody and the secondary antibody. The carrier may be washed then to remove non-bound secondary antibody. Finally, the presence of the autoantibody is detected by checking whether the secondary antibody may be detected.

In a preferred embodiment, the method is used more than once to examine samples from the same patient, preferably on different days. For example, the presence or absence of antibodies may be detected on a daily basis over one or two weeks. In a preferred embodiment, at least 2, 3, 4, 5, 6, 7, 8, 9 or 10 samples are examined on different days.

The method and reagents according to the present invention may also be used for screening the potency and the usefulness of an antiviral drug.

The method and reagents according to the present invention may also be used for screening whether donated blood is contaminated with coronavirus.

The antibody to be detected binds preferably specifically to SEQ ID NO1. Specific binding preferably means that the binding reaction is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7.

The teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to or a polypeptide comprising said fragment, more specifically to one or more amino acid or nucleic acid sequences which are, relative to the full-length sequence, truncated at one or both termini by one or more amino acids. Such a fragment comprises or encodes for a peptide having at least 10, 15, 25, 50, 75, 100, 150, 200, 250, 300, 400, 500 or 600 successive amino acids of the original sequence or for a variant thereof. For example, SEQ ID NO12 is an exemplary fragment that may be used.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

SARS-CoV-2-related publications on specific amino acid sequences such as Beal et al. may aid the skilled one in designing variants (Beal, J., Mitechell, T., Wyschogrod, W., Manthey, J, and Clore, A. (2020) Highly Distinguished Amino Acid Sequences of 2019-nCoV (Wuhan Coronavirus) doi: https://doi.org/10.1101/2020.01.31.929497, as well as publications relating to SARS-CoV, for example Hua, R., Zhou, Y., Wang, Y., Hua, Y and Tong, T. (2004) Identification of two antigenic epitopes on SARS-CoV spike protein, BBR 319, 929-935, wherein homologous epitopes may be found and SARS-CoV-2 epitopes be identified on account of their homology. For example, possible epitopes may be derived from SEQ ID NO5.

In a preferred embodiment, variants may, in addition, comprise chemical modifications, for example labels such as isotopic labels or detectable labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof, for example artificial linkers, affinity tags, other antigens and the like. For example, SEQ ID NO3 is a fusion protein according to the present invention.

The variant of the polypeptide has biological activity. In a preferred embodiment such biological activity is the ability to bind to the respective antibody. In a preferred embodiment it comprises an epitope having the ability or has itself the ability to bind to an antibody to SEQ ID NO1, preferably an IgA class antibody to SEQ ID NO1, preferably from a sample from a patient suffering from SARS-CoV-2, wherein more preferably the epitope comprises a sequence comprising at least 5, 6, 7 or 8 amino acid residues. More preferably, it does not bind specifically to homologues of SEQ ID NO1 from other coronaviruses, preferably from the group comprising MERS (SEQ ID NO6), NL63 (SEQ ID NO10), 229E (SEQ ID NO7), OC43 (SEQ ID NO8) and HKU1 (SEQ ID NO9), more preferably from the group comprising SARS-CoV-1 (SEQ ID NO11), MERS NL63, 229E, OC43 and HKU1.

The detection of the antibody or complex for the prognosis, diagnosis, methods or kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassays, enzyme immunoassays such as colorimetric assays, chemiluminscence immunoassays and immunofluorescence techniques. In a preferred embodiment, the complex is detected using a method selected from the group comprising immunodiffusion techniques, immunoelectrophoretic techniques, light scattering immunoassays, agglutination techniques, labeled immunoassays from the group comprising radiolabeled immunoassays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. Preferably the test format is an ELISA and a microtiter plate comprising wells is used as a diagnostically useful carrier.

In a preferred embodiment, a secondary antibody is an antibody binding to all antibodies from an antibody class, preferably a human antibody class, preferably IgA and/or IgG and/or IgM antibodies, preferably IgA. Secondary antibodies typically recognize the constant domain of said class. A wide range of them is commercially available.

According to the present invention, the SARS-CoV-2 infection may be detected at increased sensitivity at an early stage, preferably 5 or fewer days after the onset of disease symptoms.

The diagnostically useful carrier is preferably selected from the group comprising a glass slide, preferably for microscopy, a biochip, a microtiter plate, a lateral flow device, a test strip, a membrane, preferably a line blot, a chromatography column and a bead, preferably a microtiter plate.

According to the present invention, a polypeptide, preferably the polypeptide comprising SEQ ID NO1 or a variant thereof may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective liquid consists of said polypeptide as judged by SDS polyacrylamide gel electrophoresis followed by Coomassie blue staining and visual inspection. Preferably any polypeptide on a carrier used as a means to capture an antibody is pure.

A secondary antibody comprising a detectable label may be used to detect IgA and/or IgG and/or IgM, preferably IgA class antibodies to SEQ ID NO1. In a preferred embodiment, the label is selected from the group comprising a fluorescent, a radioactive or an enzymatically active label, preferably one catalyzing a colorimetric reaction. FITC or another fluorescein derivative may be used as a fluorescent label. A protein having peroxidase activity may be used as an enzymatically active label. Preferably the secondary antibody recognizes mammalian, more preferably human antibodies. Preferably the secondary antibody is a monoclonal antibody.

In a preferred embodiment, a kit according to the present invention comprises a polypeptide comprising SEQ ID NO1 or a variant thereof, preferably coated to a diagnostically useful carrier, more preferably a microtiter plate, and one or more, preferably all reagents from the group comprising a calibrator, a positive control, a negative control, a wash buffer, a means for detecting the presence of antibody to SEQ ID NO1, preferably an IgA class antibody, preferably a secondary antibody binding specifically antibodies, more preferably IgA class antibodies, wherein the secondary antibody may comprise a detectable label, a sample buffer, a detection solution, preferably a chromogen/substrate solution, a stop solution and a protective foil.

In a preferred embodiment, a calibrator is a reagent that binds to a polypeptide comprising SEQ ID NO1 or a variant thereof and is preferably recognized by secondary antibodies recognizing IgA class antibodies. The calibrator may be an IgA antibody to SEQ ID NO1. In a preferred embodiment, a positive control is a solution comprising a compound such as antibody to SEQ ID NO1, preferably from the group comprising IgA, IgG and IgM class antibodies, more preferably IgA, from the sample of a patient suffering from SARS-coV-2 at an amount that a positive result is obtained using the method according to the present invention. A negative control is a reagent that lacks such a compound and could comprise serum from a healthy person. A wash buffer may be used to wash the microtiter plate after the incubation to remove unspecific antibodies and could be PBS. The means for detecting the presence of an antibody could be a secondary antibody binding to the antibody class to be detected, preferably human IgA class antibodies, and is labeled, preferably with an enzyme, more preferably with an enzyme having peroxidase activity. The sample buffer may be used to dilute patient sample and may be PBS. The detection solution may yield a signal in the presence of the labeled secondary antibody and is preferably a color-developing solution and more preferably 3,3',5, 5' tetramethylbenzidine/H2O2. The stop solution may be added to a reaction to stop the reaction of the detection solution and may comprise a strong acid, preferably 0.5 M sulphuric acid. The protective foil may be place on top of the microtiter plate to avoid evaporation.

The present invention comprises a range of novel nucleic acid and polypeptide sequences, more specifically 
SEQ ID NO1 (SARS-CoV-2 S1 Spike-Protein)
VNLTTRTQLPPAYTNSFTRGVYYPDKVFRSSVLHSTQDLFLPFFSNVTWFHAIHVSGTNGT
KRFDNPVLPFNDGVYFASTEKSNIIRGWIFGTTLDSKTQSLLIVNNATNVVIKVCEFQFCNDP
FLGVYYHKNNKSWMESEFRVYSSANNCTFEYVSQPFLMDLEGKQGNFKNLREFVFKNIDG
YFKIYSKHTPINLVRDLPQGFSALEPLVDLPIGINITRFQTLLALHRSYLTPGDSSSGWTAGAA
AYYVGYLQPRTFLLKYNENGTITDAVDCALDPLSETKCTLKSFTVEKGIYQTSNFRVQPTESI
VRFPNITNLCPFGEVFNATRFASVYAWNRKRISNCVADYSVLYNSASFSTFKCYGVSPTKLN
DLCFTNVYADSFVIRGDEVRQIAPGQTGKIADYNYKLPDDFTGCVIAWNSNNLDSKVGGNY
NYLYRLFRKSNLKPFERDISTEIYQAGSTPCNGVEGFNCYFPLQSYGFQPTNGVGYQPYRV
VVLSFELLHAPATVCGPKKSTNLVKNKCVNFNFNGLTGTGVLTESNKKFLPFQQFGRDIAD
TTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRAR
 
SEQ ID NO2 (SARS-CoV-2 Spike-Protein, C-terminally his-tagged, as expressed in
cells for the example)
MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNATN
VVIKVCEFQFCNDPFLGVYYHKNNKSWMESEFRVYSSANNCTFEYVSQPFLMDLEGKQGN
FKNLREFVFKNIDGYFKIYSKHTPINLVRDLPQGFSALEPLVDLPIGINITRFQTLLALHRSYLT
PGDSSSGWTAGAAAYYVGYLQPRTFLLKYNENGTITDAVDCALDPLSETKCTLKSFTVEKG
IYQTSNFRVQPTESIVRFPNITNLCPFGEVFNATRFASVYAWNRKRISNCVADYSVLYNSAS
FSTFKCYGVSPTKLNDLCFTNVYADSFVIRGDEVRQIAPGQTGKIADYNYKLPDDFTGCVIA
WNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESN
KKFLPFQQFGRDIADTTDAVRDPQTLEILDITPCSFGGVSVITPGTNTSNQVAVLYQDVNCT
EVPVAIHADQLTPTWRVYSTGSNVFQTRAGCLIGAEHVNNSYECDIPIGAGICASYQTQTNS
PRRARLEHHHHHHHH
 
SEQ ID NO3 (SARS-CoV-2 Spike-Protein, C-terminally his-tagged, as after cleavage of
signal peptide, used in the examples)
VNLTTRTQLPPAYTNSFTRGVYYPDKVFRSSVLHSTQDLFLPFFSNVTWFHAIHVSGTNGT
KRFDNPVLPFNDGVYFASTEKSNIIRGWIFGTTLDSKTQSLLIVNNATNVVIKVCEFQFCNDP
FLGVYYHKNNKSWMESEFRVYSSANNCTFEYVSQPFLMDLEGKQGNFKNLREFVFKNIDG
YFKIYSKHTPINLVRDLPQGFSALEPLVDLPIGINITRFQTLLALHRSYLTPGDSSSGWTAGAA
AYYVGYLQPRTFLLKYNENGTITDAVDCALDPLSETKCTLKSFTVEKGIYQTSNFRVQPTESI
VRFPNITNLCPFGEVFNATRFASVYAWNRKRISNCVADYSVLYNSASFSTFKCYGVSPTKLN
DLCFTNVYADSFVIRGDEVRQIAPGQTGKIADYNYKLPDDFTGCVIAWNSNNLDSKVGGNY
NYLYRLFRKSNLKPFERDISTEIYQAGSTPCNGVEGFNCYFPLQSYGFQPTNGVGYQPYRV
 
VVLSFELLHAPATVCGPKKSTNLVKNKCVNFNFNGLTGTGVLTESNKKFLPFQQFGRDIAD
TTDAVRDPQTLEILDITPCSFGGVSVITPGTNTSNQVAVLYQDVNCTEVPVAIHADQLTPTW
RVYSTGSNVFQTRAGCLIGAEHVNNSYECDIPIGAGICASYQTQTNSPRRARLEHHHHHH
H
 
SEQ ID NO4 (SARS-CoV-2 Spike-Protein, nucleotide sequence encoding SEQ ID NO2)
ATGTTCGTATTCCTTGTTCTGCTGCCTTTGGTTAGCAGTCAGTGTGTCAACCTGACAACT
CGCACGCAACTGCCGCCAGCTTACACCAACTCTTTCACAAGAGGCGTCTACTACCCGG
ACAAAGTGTTTCGCTCATCAGTGCTGCACTCTACACAAGATTTGTTTCTGCCATTCTTCT
CTAACGTAACCTGGTTTCACGCGATTCATGTGTCTGGGACAAATGGGACCAAGCGCTTC
GACAACCCCGTGCTGCCATTCAATGACGGGGTGTATTTTGCCTCCACCGAGAAATCCA
ATATCATCCGAGGATGGATTTTCGGTACTACGCTGGACTCTAAAACGCAGTCTCTCTTG
ATCGTTAATAACGCCACAAATGTTGTCATTAAGGTGTGCGAGTTTCAGTTCTGTAATGAT
CCCTTTCTGGGTGTGTATTACCACAAGAATAACAAGTCATGGATGGAAAGCGAGTTTCG
CGTGTACTCAAGTGCCAATAACTGCACATTCGAGTATGTGTCCCAGCCTTTCCTGATGG
ATCTCGAAGGCAAACAGGGGAACTTCAAGAATCTGCGCGAGTTCGTGTTTAAGAACATC
GACGGTTATTTCAAGATCTACAGCAAACATACACCCATTAACCTGGTCAGGGATCTCCC
TCAGGGATTCTCCGCCCTGGAACCCTTGGTGGACTTGCCCATTGGGATTAACATCACTA
GATTCCAGACCCTGCTGGCCCTTCACCGTTCCTATCTTACTCCTGGCGACAGTAGCAGT
GGATGGACCGCAGGAGCAGCCGCTTACTATGTAGGCTATCTGCAGCCACGGACCTTCC
TCCTCAAGTACAATGAAAATGGTACCATAACTGATGCTGTGGACTGCGCTCTGGATCCA
CTCTCCGAAACTAAATGCACCCTTAAAAGCTTCACGGTCGAAAAGGGAATCTACCAGAC
AAGTAACTTTCGGGTACAACCCACTGAGTCCATCGTGCGGTTTCCTAACATCACAAATC
TCTGCCCCTTTGGTGAAGTGTTTAACGCCACTAGGTTCGCTTCTGTTTATGCGTGGAAT
CGGAAGAGGATTTCCAATTGCGTGGCAGACTACTCTGTCCTGTATAATAGCGCTAGCTT
CAGCACCTTCAAATGTTACGGGGTAAGCCCAACTAAACTGAACGACCTCTGTTTTACCA
ACGTGTATGCCGATAGCTTTGTCATACGAGGAGATGAGGTTCGTCAGATTGCTCCTGG
CCAAACGGGGAAAATCGCAGACTACAACTACAAGCTTCCCGACGACTTCACAGGATGC
GTGATCGCGTGGAACTCAAATAATCTGGATAGCAAGGTTGGTGGCAATTATAACTACCT
GTATCGACTGTTCAGGAAAAGCAACCTCAAACCCTTTGAGCGCGACATCAGCACCGAG
ATATACCAAGCCGGTTCAACACCTTGCAATGGGGTGGAAGGGTTTAACTGCTATTTCCC
ACTTCAGAGCTATGGGTTTCAGCCAACCAATGGAGTCGGCTACCAGCCCTATCGGGTG
GTAGTCCTGTCCTTTGAGCTGTTGCATGCGCCTGCCACAGTCTGTGGCCCTAAGAAGA
GTACGAATCTGGTGAAGAACAAGTGCGTCAACTTCAATTTTAACGGCTTGACTGGAACA
GGAGTTCTGACCGAGTCCAACAAGAAATTCCTTCCTTTTCAGCAGTTTGGAAGGGATAT
AGCCGACACTACCGATGCCGTTCGGGATCCACAGACACTGGAGATTCTGGACATTACT
CCGTGCTCATTTGGCGGTGTATCTGTCATCACACCTGGGACCAATACCTCAAATCAGGT
GGCTGTGCTCTACCAGGATGTGAATTGTACCGAAGTTCCAGTGGCAATTCATGCCGATC
 
AACTGACTCCCACCTGGAGAGTGTACAGTACTGGCAGTAACGTGTTTCAGACAAGAGC
TGGCTGTCTCATAGGCGCAGAACACGTCAACAACAGCTATGAGTGTGACATTCCGATC
GGCGCAGGCATCTGTGCATCCTACCAGACGCAAACCAACTCTCCCAGAAGAGCCAGGC
TCGAGCACCACCATCACCATCACCATCACTAA
 **SEQ ID NO5 (possible epitope)**
NLKPFERDISTE

SEQ ID NO6 (S1[MERS_CoV])
YVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKI
YPAFMLGSSVGNFSDGKMGRFFNHTLVLLPDGCGTLLRAFYCILEPRSGNHCPAGNSYTSF
ATYHTPATDCSDGNYNRNASLNSFKEYFNLRNCTFMYTYNITEDEILEWFGITQTAQGVHLF
SSRYVDLYGGNMFQFATLPVYDTIKYYSIIPHSIRSIQSDRKAWAAFYVYKLQPLTFLLDFSV
DGYIRRAIDCGFNDLSQLHCSYESFDVESGVYSVSSFEAKPSGSVVEQAEGVECDFSPLLS
GTPPQVYNFKRLVFTNCNYNLTKLLSLFSVNDFTCSQISPAAIASNCYSSLILDYFSYPLSMK
SDLSVSSAGPISQFNYKQSFSNPTCLILATVPHNLTTITKPLKYSYINKCSRLLSDDRTEVPQL
VNANQYSPCVSIVPSTVWEDGDYYRKQLSPLEGGGWLVASGSTVAMTEQLQMGFGITVQ
YGTDTNSVCPKLEFANDTKIASQLGNCVEYSLYGVSGRGVFQNCTAVGVRQQRFVYDAYQ
NLVGYYSDDGNYYCLRACVSVPVSVIYDKETKTHATLFGSVACEHISSTMSQYSRSTRSML
KRRDSTYGPLQTPVGCVLGLVNSSLFVEDCKLPLGQSLCALPDTPSTLTPRSVR

SEQ ID NO7 (S1[HCoV-229E])
CQTTNGLNTSYSVCNGCVGYSENVFAVESGGYIPSDFAFNNWFLLTNTSSVVDGVVRSFQ
PLLLNCLWSVSGLRFTTGFVYFNGTGRGDCKGFSSDVLSDVIRYNLNFEENLRRGTILFKTS
YGVVVFYCTNNTLVSGDAHIPFGTVLGNFYCFVNTTIGNETTSAFVGALPKTVREFVISRTG
HFYINGYRYFTLGNVEAVNFNVTTAETTDFCTVALASYADVLVNVSQTSIANIIYCNSVINRLR
CDQLSFDVPDGFYSTSPIQSVELPVSIVSLPVYHKHTFIVLYVDFKPQSGGGKCFNCYPAGV
NITLANFNETKGPLCVDTSHFTTKYVAVYANVGRWSASINTGNCPFSFGKVNNFVKFGSVC
FSLKDIPGGCAMPIVANWAYSKYYTIGSLYVSWSDGDGITGVPQPVEGVSSFMNVTLDKCT
KYNIYDVSGVGVIRVSNDTFLNGITYTSTSGNLLGFKDVTKGTIYSITPCNPPDQLVVYQQAV
VGAMLSENFTSYGFSNVVELPKFFYA

SEQ ID NO8 (S1[HCoV-OC43])
AVIGDLKCTSDNINDKDTGPPPISTDTVDVTNGLGTYYVLDRVYLNTTLFLNGYYPTSGSTYR
NMALKGSVLLSRLWFKPPFLSDFINGIFAKVKNTKVIKDRVMYSEFPAITIGSTFVNTSYSVVV
QPRTINSTQDGDNKLQGLLEVSVCQYNMCEYPQTICHPNLGNHRKELWHLDTGVVSCLYK
RNFTYDVNADYLYFHFYQEGGTFYAYFTDTGVVTKFLFNVYLGMALSHYYVMPLTCNSKLT

LEYWVTPLTSRQYLLAFNQDGIIFNAEDCMSDFMSEIKCKTQSIAPPTGVYELNGYTVQPIAD
VYRRKPNLPNCNIEAWLNDKSVPSPLNWERKTFSNCNFNMSSLMSFIQADSFTCNNIDAAK
IYGMCFSSITIDKFAIPNGRKVDLQLGNLGYLQSFNYRIDTTATSCQLYYNLPAANVSVSRFN
PSTWNKRFGFIEDSVFKPRPAGVLTNHDVVYAQHCFKAPKNFCPCKLNGSCVGSGPGKN
NGIGTCPAGTNYLTCDNLCTPDPITFTGTYKCPQTKSLVGIGEHCSGLAVKSDYCGGNSCT
CRPQAFLGWSADSCLQGDKCNIFANFILHDVNSGLTCSTDLQKANTDIILGVCVNYDLYGIL
GQGIFVEVNATYYNSWQNLLYDSNGNLYGFRDYIINRTFMIRSCYSGRVSAAFHANSSEPAL
LFRNIKCNYVFNNSLTRQLQPINYFDSYLGCVVNAYNSTAISVQTCDLTVGSGYCVDYSKNR
RSRG

SEQ ID NO9 (S1[HCoV-HKU1])
AVIGDFNCTNSFINDYNKTIPRISEDVVDVSLGLGTYYVLNRVYLNTTLLFTGYFPKSGANFR
DLALKGSIYLSTLWYKPPFLSDFNNGIFSKVKNTKLYVNNTLYSEFSTIVIGSVFVNTSYTIVV
QPHNGILEITACQYTMCEYPHTVCKSKGSIRNESWHIDSSEPLCLFKKNFTYNVSADWLYFH
FYQERGVFYAYYADVGMPTTFLFSLYLGTILSHYYVMPLTCNAISSNTDNETLEYWVTPLSR
RQYLLNFDEHGVITNAVDCSSSFLSEIQCKTQSFAPNTGVYDLSGFTVKPVATVYRRIPNLP
DCDIDNWLNNVSVPSPLNWERRIFSNCNFNLSTLLRLVHVDSFSCNNLDKSKIFGSCFNSIT
VDKFAIPNRRRDDLQLGSSGFLQSSNYKIDISSSSCQLYYSLPLVNVTINNFNPSSWNRRYG
FGSFNLSSYDVVYSDHCFSVNSDFCPCADPSVVNSCAKSKPPSAICPAGTKYRHCDLDTTL
YVKNWCRCSCLPDPISTYSPNTCPQKKVVVGIGEHCPGLGINEEKCGTQLNHSSCFCSPDA
FLGWSFDSCISNNRCNIFSNFIFNGINSGTTCSNDLLYSNTEISTGVCVNYDLYGITGQGIFKE
VSAAYYNNWQNLLYDSNGNIIGFKDFLTNKTYTILPCYSGRVSAAFYQNSSSPALLYRNLKC
SYVLNNISFISQPFYFDSYLGCVLNAVNLTSYSVSSCDLRMGSGFCIDYALPSSRRKRR

SEQ ID NO10 (S1[HCoV-NL63])
FFTCNSNANLSMLQLGVPDNSSTIVTGLLPTHWFCANQSTSVYSANGFFYIDVGNHRSAFA
LHTGYYDANQYYIYVTNEIGLNASVTLKICKFSRNTTFDFLSNASSSFDCIVNLLFTEQLGAPL
GITISGETVRLHLYNVTRTFYVPAAYKLTKLSVKCYFNYSCVFSVVNATVTVNVTTHNGRVV
NYTVCDDCNGYTDNIFSVQQDGRIPNGFPFNNWFLLTNGSTLVDGVSRLYQPLRLTCLWP
VPGLKSSTGFVYFNATGSDVNCNGYQHNSVVDVMRYNLNFSANSLDNLKSGVIVFKTLQY
DVLFYCSNSSSGVLDTTIPFGPSSQPYYCFINSTINTTHVSTFVGILPPTVREIVVARTGQFYI
NGFKYFDLGFIEAVNFNVTTASATDFWTVAFATFVDVLVNVSATNIQNLLYCDSPFEKLQCE
HLQFGLQDGFYSANFLDDNVLPETYVALPIYYQHTDINFTATASFGGSCYVCKPHQVNISLN
GNTSVCVRTSHFSIRYIYNRVKSGSPGDSSWHIYLKSGTCPFSFSKLNNFQKFKTICFSTVE
VPGSCNFPLEATWHYTSYTIVGALYVTWSEGNSITGVPYPVSGIREFSNLVLNNCTKYNIYD
YVGTGIIRSSNQSLAGGITYVSNSGNLLGFKNVSTGNIFIVTPCNQPDQVAVYQQSIIGAMTA
VNESRYGLQNLLQLPNFYYV

SEQ ID NO11 (S1[SARS_CoV])
SGSDLDRCTTFDDVQAPNYTQHTSSMRGVYYPDEIFRSDTLYLTQDLFLPFYSNVTGFHTIN
HTFGNPVIPFKDGIYFAATEKSNVVRGWVFGSTMNNKSQSVIIINNSTNVVIRACNFELCDNP
FFAVSKPMGTQTHTMIFDNAFNCTFEYISDAFSLDVSEKSGNFKHLREFVFKNKDGFLYVYK
GYQPIDVVRDLPSGFNTLKPIFKLPLGINITNFRAILTAFSPAQDIWGTSAAAYFVGYLKPTTF
MLKYDENGTITDAVDCSQNPLAELKCSVKSFEIDKGIYQTSNFRVVPSGDVVRFPNITNLCP
FGEVFNATKFPSVYAWERKKISNCVADYSVLYNSTFFSTFKCYGVSATKLNDLCFSNVYAD
SFVVKGDDVRQIAPGQTGVIADYNYKLPDDFMGCVLAWNTRNIDATSTGNYNYKYRYLRHG
KLRPFERDISNVPFSPDGKPCTPPALNCYWPLNDYGFYTTTGIGYQPYRVVVLSFELLNAPA
TVCGPKLSTDLIKNQCVNFNFNGLTGTGVLTPSSKRFQPFQQFGRDVSDFTDSVRDPKTSE
ILDISPCSFGGVSVITPGTNASSEVAVLYQDVNCTDVSTAIHADQLTPAWRIYSTGNNVFQT
QAGCLIGAEHVDTSYECDIPIGAGICASYHTVSLLRL

SEQ ID NO12 (fragment of SEQ ID NO1)
NSFTRGVYYPDKVFRSSVLHSTQDLFLPFFSNVTWFHAIHVSGTNGTKRFDNPVLPFNDGV
YFASTEKSNIIRGWIFGTTLDSKTQSLLIVNNATNVVIKVCEFQFCNDPFLGVYYHKNNKSW
MESEFRVYSSANNCTFEYVSQPFLMDLEGKQGNFKNLREFVFKNIDGYFKIYSKHTPINLVR
DLPQGFSALEPLVDLPIGINITRFQTLLALHRSYLTPGDSSSGWTAGAAAYYVGYLQPRTFLL
KYNENGTITDAVDCALDPLSETKCTLKSFTVEKGIYQTSNFRVQPTESIVRFPNITNLCPFGE
VFNATRFASVYAWNRKRISNCVADYSVLYNSASFSTFKCYGVSPTKLNDLCFTNVYADSFVI
RGDEVRQIAPGQTGKIADYNYKLPDDFTGCVIAWNSNNLDSKVGGNYNYLYRLFRKSNLKP
FERDISTEIYQAGSTPCNGVEGFNCYFPLQSYGFQPTNGVGYQPYRVVVLSFELLHAPATV
CGPKKSTNLVKNKCVNFNFNGLTGTGVLTESNKKFLPFQQFGRDIADTTDAVRDPQTLEILD
ITPCSFGGVSVITPGTNTSNQVAVLYQDVNCTEVPVAIHADQLTPTWRVYSTGSNVFQTRA
GCLIGAEHVNNSYECDIPIGAGICASYQTQT

The present invention is further illustrated by the following examples, sequences and figures from which further features, embodiments, aspects and advantages of the present invention may be taken. All methods and materials similar or equivalent to those described herein can be used in the practice or testing of the present invention, with suitable methods and materials being described herein. All publications, patent applications, patents, and other references mentioned herein are incorporated by reference in their entirety.

### Examples

### Samples

Eight samples from patients tested SARS-coV-2 positive by PCR as described by Corman *et al.* (Corman et al. (2020) Diagnostic detection of 2019-nCoV by real-time RT-PCR, https://www.who.int/docs/default-source/coronaviruse/protocol-v2-1.pdf?sfvrsn=a9ef618c 2), which were obtained 6 to 14 days after the infection and 14 samples from such patients obtained at an earlier time point after the infection were available.

In addition, a range of samples containing various coronaviruses was available, including 18 samples from patients infected with MERS, three samples from patients infected with SARS-CoV-1, four patients with NL63, three patients with 229E, six patients with OC43 and three patients with HKU1.

### Preparation of microtiter plates coated with antigen

SEQID NO2 was expressed in HEK293T cells using standard cloning of SEQ ID NO4 into the pTriEx-1 plasmid with an artificial signal sequence and a C-terminal His tag, resulting in the expression of SEQ ID NO2 and, after removal of the signal peptide, SEQ ID NO3. Transfected cells were cultures at 37°C and 8.5% CO2 in Dulbecco's modified eagle's medium with 10% fetal calf serum, 100 U/ml penicillin and 0.1 mg/ml streptomycin for three to five days. Cells were harvested, resuspended in 20 mM Tris-HCl pH 7.4, 10% (w/v) sucrose, 5 mM EDTA, 1 mM PMSF and stored at -80°C until further use.

To prepare SEQ ID NO3, cell culture supernatant was adjusted to 5 mmol/l tris chloride pH 8.0, 164 mmol/l sodium chloride, 50 mmol/l magnesium chloride, 20 mmol/l imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/l tris chloride pH 8.0, 300 mmol/l sodium chloride, 20 mmol/l imidazole and eluted by increasing the imidazole concentration to 150 mmol/l. All fractions containing SEQ ID NO3 were pooled and concentrated by ultrafiltration (VivaSpin, Sartorius, Göttingen, Germany). The final preparation was stored at -80°C until further use.

The final protein preparation of SEQ ID NO3 was treated with or without 16 mmol/l dithio-treitol and incubated at 70°C or at room temperature for 10 minutes, followed by SDS gel electrophoresis and Coomassie staining.

Protein identity was verified by mass spectrometry.

For use in microtiter ELISA the purified protein was diluted in PBS to final concentrations of approximately 1.5 µg/ml and used to coat ELISA microtiter plates (Nunc, Roskilde, Denmark) overnight.

### Experimental procedure

Samples were diluted 1:101 in IgG sample buffer, applied to microtiter plates and incubated as described for commercial EUROIMMUN ELISA Test-Kits, using reagents commercially available (e.g. EI 2260-9601 G/A). The manual of EI 2260-9601 G/A was followed. In brief: 60 min at 37 °C; 3 washing steps using washing buffer; addition of 100 µl of peroxidase-labelled anti-human IgG conjugate (rabbit) or anti-human IgA conjugate (rabbit) per well; incubation for 30 min at 37 °C; 3 washing steps using EUROIMMUN washing buffer; addition of 100 µl of chromogen/substrate solution (TMB/H₂O₂) per well; incubation for 30 min at room temperature; addition of 100 µl stop-solution (0.5 M sulfuric acid); measurement of optical density at 450 nm against 630 nm as a reference.

Calibration was carried out using commercially available calibrators (product number EI 2606-9601 A, EUROIMMUN Medizinische Labordiagnostika AG). A ratio was calculated by dividing extinction of the control or patient sample by the extinction of the calibrator. Results below 0.8 were considered negative, results between 0.8 and 1.1 borderline, and results of more than 1.1 positive.

### Results

The primary data are shown in Table 1:

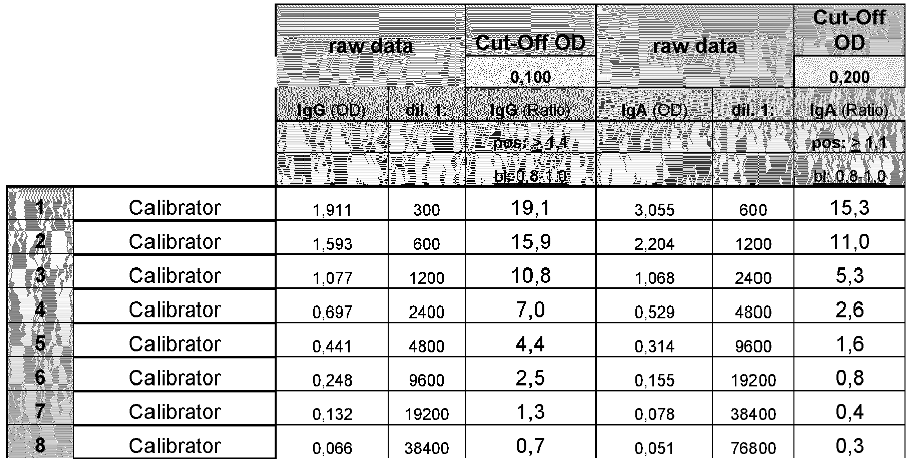

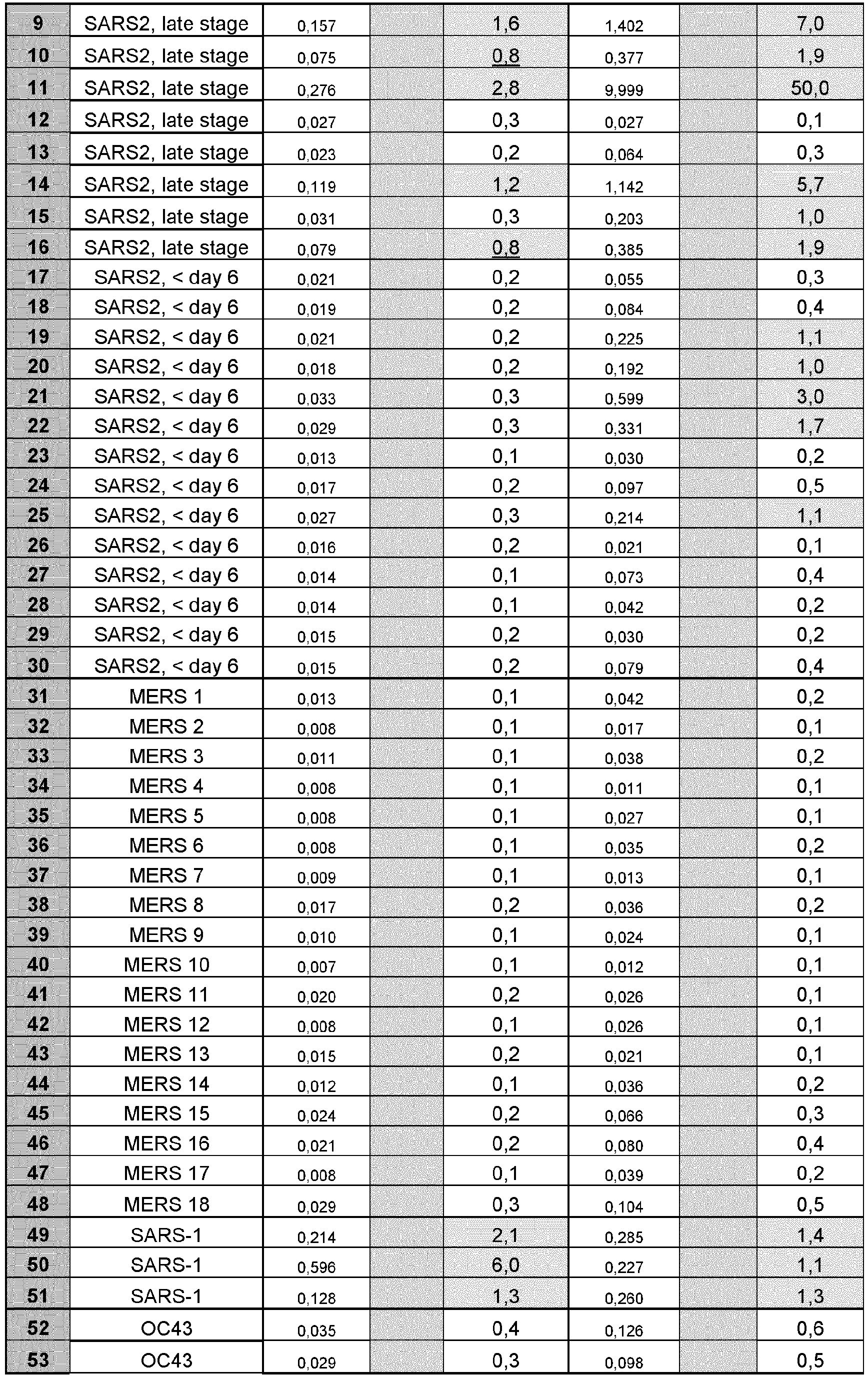

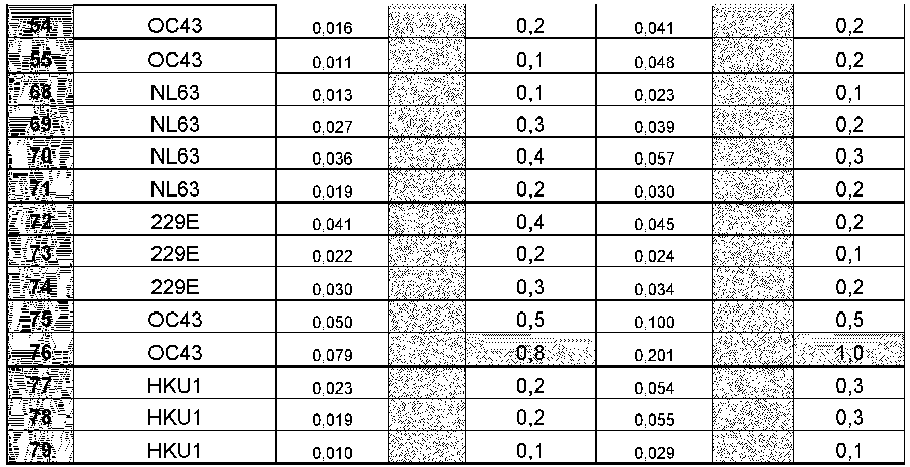

### Conclusions

The results show that antibodies to SEQ ID NO1 may be used to diagnose an SARS-CoV-2 infection in samples from human patients.

Comparison of the data obtained with secondary antibodies recognizing IgG and IgA class antibodies shows that the detection of IgA antibodies is more sensitive: 4/14 patient samples taken at an earlier stage of the infection, before six days post onset of illness, could be correctly identified as positive when IgA class antibodies were detected, while the detection of IgG in the same samples gave negative results.

Both assays showed cross-reactivity with samples from SARS-CoV-1 patients, but virtually none of the samples from patients infected with MERS, NL63, 229E, OC43 and HKU1.

## Claims

1. A method for diagnosing a SARS-CoV-2 infection comprising the step of detecting the presence or absence of an antibody to SEQ ID NO1, preferably IgA class antibody, in a sample from a subject.

2. A method for the differential diagnosis of a coronavirus infection, preferably for distinguishing between a SARS-CoV; preferably SARS-CoV-2; MERS, NL63, 229E, OC43 and HKU1 infection, comprising the step of detecting the presence or absence of an antibody to SEQ ID NO1 in a sample from a subject, preferably an IgA and/or IgG class antibody, more preferably an IgA class antibody.

3. The method according to any of claims 1 or 2, wherein the presence of an IgG and/or IgM class antibody to SEQ ID NO1 is detected in addition to an IgA class antibody to SEQ ID NO1.

4. The method according to any of claims 1 to 3, wherein the sample is a blood sample, preferably selected from the group comprising whole blood, serum or plasma.

5. The method according to any of claims 1 to 4, wherein an IgA class antibody to SEQ ID NO1 is detected using a labeled secondary antibody, preferably binding to IgA class antibodies.

6. The method according to any of claims 1 to 5, wherein the IgA antibody is detected using a method selected from the group comprising colorimetry, immunofluorescence, detection of enzymatic activity, chemiluminscence and radioactivity.

7. The method according to any of claims 1 to 6, wherein the infection is detected at an early stage.

8. A use of an antibody to SEQ ID NO1, preferably IgA class antibody, for diagnosing a SARS-CoV-2 infection or for the differential diagnosis of a coronavirus infection, preferably for distinguishing between a SARS-CoV, preferably SARS-CoV-2, MERS and NL63, 229E, OC43 and HKU1 infection.

9. The use according to claim 8, wherein the use is for the early diagnosis of a SARS-CoV-2 infection.

10. A kit comprising a polypeptide comprising SEQ ID NO1 or a variant thereof, preferably coated to a diagnostically useful carrier and one or more, preferably all reagents from the group comprising an antibody to SEQ ID NO1, a washing buffer, a means for detecting the presence of antibody to SEQ ID NO1, preferably an IgA class antibody, preferably a secondary antibody binding specifically to IgA class antibodies, preferably comprising a detectable label, and a dilution buffer.

11. The kit according to claim 10, wherein the diagnostically useful carrier is selected from the group comprising a bead, preferably a paramagnetic bead, a test strip, a microtiter plate, a membrane, preferably from the group comprising western blot, line blot and dot blot, a lateral flow device, a glass surface, a slide, a microarray and a biochip and is preferably a microtiter plate.

12. The kit according to claim 10 or 11, wherein the kit comprises two or more, preferably three or more calibrators.

13. The kit according to claim 12, wherein each calibrator is a recombinant antibody binding to SEQ ID NO1 which is preferably recognized by a secondary antibody binding to IgA class antibodies.

14. A use of a polypeptide comprising SEQ ID NO1 or a variant thereof or an antibody to SEQ ID NO1, preferably an IgA class antibody, for the manufacture of a diagnostic kit.

15. A use of a recombinant antibody binding to SEQ ID NO1 which is preferably recognized by a secondary antibody binding to IgA class antibodies as a calibrator for the early diagnosis of a SARS-CoV-2 infection.


SEQUENCE LISTING

<110>  EUROIMMUN Medizinische Labordiagnostika AG

<120>  A method and reagents for the diagnosis of SARS-CoV-2

<130>  20PP007EP

<160>  12

<170>  PatentIn version 3.5

<210>  1
<211>  670
<212>  PRT
<213>  Artificial Sequence

<220>
<223>  SARS-CoV-2 S1 Spike-Protein

<400>  1

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu

                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
        420                 425                 430

Asn Tyr Asn Tyr Leu Tyr Arg Leu Phe Arg Lys Ser Asn Leu Lys Pro
    435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr
465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg
            660                 665                 670

<210>  2
<211>  695
<212>  PRT
<213>  Artificial Sequence

<220>
<223>  SARS-CoV-2 Spike-Protein, C-terminally his-tagged

<400>  2

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Asn Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn
        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Leu Glu His
        675                 680                 685

His His His His His His His
    690                 695

<210>  3
<211>  680

<212>  PRT
<213>  Artificial Sequence

<220>
<223>  SARS-CoV-2 Spike-Protein, C-terminally his-tagged, as after
       cleavage of signal peptide

<400>  3

Val Asn Leu Thr Thr Arg Thr Gln Leu Pro Pro Ala Tyr Thr Asn Ser
1               5                   10                  15

Phe Thr Arg Gly Val Tyr Tyr Pro Asp Lys Val Phe Arg Ser Ser Val
            20                  25                  30

Leu His Ser Thr Gln Asp Leu Phe Leu Pro Phe Phe Ser Asn Val Thr
        35                  40                  45

Trp Phe His Ala Ile His Val Ser Gly Thr Asn Gly Thr Lys Arg Phe
    50                  55                  60

Asp Asn Pro Val Leu Pro Phe Asn Asp Gly Val Tyr Phe Ala Ser Thr
65                  70                  75                  80

Glu Lys Ser Asn Ile Ile Arg Gly Trp Ile Phe Gly Thr Thr Leu Asp
                85                  90                  95

Ser Lys Thr Gln Ser Leu Leu Ile Val Asn Asn Ala Thr Asn Val Val
            100                 105                 110

Ile Lys Val Cys Glu Phe Gln Phe Cys Asn Asp Pro Phe Leu Gly Val
        115                 120                 125

Tyr Tyr His Lys Asn Asn Lys Ser Trp Met Glu Ser Glu Phe Arg Val
    130                 135                 140

Tyr Ser Ser Ala Asn Asn Cys Thr Phe Glu Tyr Val Ser Gln Pro Phe
145                 150                 155                 160

Leu Met Asp Leu Glu Gly Lys Gln Gly Asn Phe Lys Asn Leu Arg Glu
                165                 170                 175

Phe Val Phe Lys Asn Ile Asp Gly Tyr Phe Lys Ile Tyr Ser Lys His
            180                 185                 190

Thr Pro Ile Asn Leu Val Arg Asp Leu Pro Gln Gly Phe Ser Ala Leu
        195                 200                 205

Glu Pro Leu Val Asp Leu Pro Ile Gly Ile Asn Ile Thr Arg Phe Gln
    210                 215                 220

Thr Leu Leu Ala Leu His Arg Ser Tyr Leu Thr Pro Gly Asp Ser Ser
225                 230                 235                 240

Ser Gly Trp Thr Ala Gly Ala Ala Ala Tyr Tyr Val Gly Tyr Leu Gln
                245                 250                 255

Pro Arg Thr Phe Leu Leu Lys Tyr Asn Glu Asn Gly Thr Ile Thr Asp
            260                 265                 270

Ala Val Asp Cys Ala Leu Asp Pro Leu Ser Glu Thr Lys Cys Thr Leu
        275                 280                 285

Lys Ser Phe Thr Val Glu Lys Gly Ile Tyr Gln Thr Ser Asn Phe Arg
    290                 295                 300

Val Gln Pro Thr Glu Ser Ile Val Arg Phe Pro Asn Ile Thr Asn Leu
305                 310                 315                 320

Cys Pro Phe Gly Glu Val Phe Asn Ala Thr Arg Phe Ala Ser Val Tyr
                325                 330                 335

Ala Trp Asn Arg Lys Arg Ile Ser Asn Cys Val Ala Asp Tyr Ser Val
            340                 345                 350

Leu Tyr Asn Ser Ala Ser Phe Ser Thr Phe Lys Cys Tyr Gly Val Ser
        355                 360                 365

Pro Thr Lys Leu Asn Asp Leu Cys Phe Thr Asn Val Tyr Ala Asp Ser
    370                 375                 380

Phe Val Ile Arg Gly Asp Glu Val Arg Gln Ile Ala Pro Gly Gln Thr
385                 390                 395                 400

Gly Lys Ile Ala Asp Tyr Asn Tyr Lys Leu Pro Asp Asp Phe Thr Gly
                405                 410                 415

Cys Val Ile Ala Trp Asn Ser Asn Asn Leu Asp Ser Lys Val Gly Gly
            420                 425                 430

Asn Tyr Asn Tyr Leu Tyr Arg Leu Phe Arg Lys Ser Asn Leu Lys Pro
        435                 440                 445

Phe Glu Arg Asp Ile Ser Thr Glu Ile Tyr Gln Ala Gly Ser Thr Pro
    450                 455                 460

Cys Asn Gly Val Glu Gly Phe Asn Cys Tyr Phe Pro Leu Gln Ser Tyr

465                 470                 475                 480

Gly Phe Gln Pro Thr Asn Gly Val Gly Tyr Gln Pro Tyr Arg Val Val
                485                 490                 495

Val Leu Ser Phe Glu Leu Leu His Ala Pro Ala Thr Val Cys Gly Pro
            500                 505                 510

Lys Lys Ser Thr Asn Leu Val Lys Asn Lys Cys Val Asn Phe Asn Phe
        515                 520                 525

Asn Gly Leu Thr Gly Thr Gly Val Leu Thr Glu Ser Asn Lys Lys Phe
    530                 535                 540

Leu Pro Phe Gln Gln Phe Gly Arg Asp Ile Ala Asp Thr Thr Asp Ala
545                 550                 555                 560

Val Arg Asp Pro Gln Thr Leu Glu Ile Leu Asp Ile Thr Pro Cys Ser
                565                 570                 575

Phe Gly Gly Val Ser Val Ile Thr Pro Gly Thr Asn Thr Ser Asn Gln
            580                 585                 590

Val Ala Val Leu Tyr Gln Asp Val Asn Cys Thr Glu Val Pro Val Ala
        595                 600                 605

Ile His Ala Asp Gln Leu Thr Pro Thr Trp Arg Val Tyr Ser Thr Gly
    610                 615                 620

Ser Asn Val Phe Gln Thr Arg Ala Gly Cys Leu Ile Gly Ala Glu His
625                 630                 635                 640

Val Asn Asn Ser Tyr Glu Cys Asp Ile Pro Ile Gly Ala Gly Ile Cys
                645                 650                 655

Ala Ser Tyr Gln Thr Gln Thr Asn Ser Pro Arg Arg Ala Arg Leu Glu
            660                 665                 670

His His His His His His His His
        675                 680

<210>  4
<211>  2088
<212>  DNA
<213>  Artificial Sequence

<220>
<223>  SARS-CoV-2 Spike-Protein, nucleotide sequence encoding SEQ ID NO2

<400> 4
atgttcgtat tccttgttct gctgcctttg gttagcagtc agtgtgtcaa cctgacaact 60

cgcacgcaac tgccgccagc ttacaccaac tctttcacaa gaggcgtcta ctacccggac 120

aaagtgtttc gctcatcagt gctgcactct acacaagatt tgtttctgcc attcttctct 180

aacgtaacct ggtttcacgc gattcatgtg tctgggacaa atgggaccaa gcgcttcgac 240

aaccccgtgc tgccattcaa tgacggggtg tattttgcct ccaccgagaa atccaatatc 300

atccgaggat ggattttcgg tactacgctg gactctaaaa cgcagtctct cttgatcgtt 360

aataacgcca caaatgttgt cattaaggtg tgcgagtttc agttctgtaa tgatcccttt 420

ctgggtgtgt attaccacaa gaataacaag tcatggatgg aaagcgagtt tcgcgtgtac 480

tcaagtgcca ataactgcac attcgagtat gtgtcccagc ctttcctgat ggatctcgaa 540

ggcaaacagg ggaacttcaa gaatctgcgc gagttcgtgt ttaagaacat cgacggttat 600

ttcaagatct acagcaaaca tacacccatt aacctggtca gggatctccc tcagggattc 660

tccgccctgg aacccttggt ggacttgccc attgggatta acatcactag attccagacc 720

ctgctggccc ttcaccgttc ctatcttact cctggcgaca gtagcagtgg atggaccgca 780

ggagcagccg cttactatgt aggctatctg cagccacgga ccttcctcct caagtacaat 840

gaaaatggta ccataactga tgctgtggac tgcgctctgg atccactctc cgaaactaaa 900

tgcaccctta aaagcttcac ggtcgaaaag ggaatctacc agacaagtaa ctttcgggta 960

caacccactg agtccatcgt gcggtttcct aacatcacaa atctctgccc ctttggtgaa 1020

gtgtttaacg ccactaggtt cgcttctgtt tatgcgtgga atcggaagag gatttccaat 1080

tgcgtggcag actactctgt cctgtataat agcgctagct tcagcacctt caaatgttac 1140

ggggtaagcc caactaaact gaacgacctc tgttttacca acgtgtatgc cgatagcttt 1200

gtcatacgag gagatgaggt tcgtcagatt gctcctggcc aaacggggaa aatcgcagac 1260

tacaactaca agcttcccga cgacttcaca ggatgcgtga tcgcgtggaa ctcaaataat 1320

ctggatagca aggttggtgg caattataac tacctgtatc gactgttcag gaaaagcaac 1380

ctcaaaccct ttgagcgcga catcagcacc gagatatacc aagccggttc aacaccttgc 1440

aatggggtgg aagggtttaa ctgctatttc ccacttcaga gctatgggtt tcagccaacc 1500

aatggagtcg gctaccagcc ctatcgggtg gtagtcctgt cctttgagct gttgcatgcg 1560

cctgccacag tctgtggccc taagaagagt acgaatctgg tgaagaacaa gtgcgtcaac 1620

ttcaatttta acggcttgac tggaacagga gttctgaccg agtccaacaa gaaattcctt 1680

ccttttcagc agtttggaag ggatatagcc gacactaccg atgccgttcg ggatccacag 1740

acactggaga ttctggacat tactccgtgc tcatttggcg gtgtatctgt catcacacct 1800

gggaccaata cctcaaatca ggtggctgtg ctctaccagg atgtgaattg taccgaagtt 1860

ccagtggcaa ttcatgccga tcaactgact cccacctgga gagtgtacag tactggcagt 1920

aacgtgtttc agacaagagc tggctgtctc ataggcgcag aacacgtcaa caacagctat 1980

gagtgtgaca ttccgatcgg cgcaggcatc tgtgcatcct accagacgca aaccaactct 2040

cccagaagag ccaggctcga gcaccaccat caccatcacc atcactaa 2088

<210> 5
<211> 12
<212> PRT
<213> Artificial Sequence

<220>
<223> possible epitope

<400> 5

Asn Leu Lys Pro Phe Glu Arg Asp Ile Ser Thr Glu
1               5                   10

<210> 6
<211> 734
<212> PRT
<213> Artificial Sequence

<220>
<223> S1 [MERS_CoV]

<400> 6

Tyr Val Asp Val Gly Pro Asp Ser Val Lys Ser Ala Cys Ile Glu Val
1               5                   10                  15

Asp Ile Gln Gln Thr Phe Phe Asp Lys Thr Trp Pro Arg Pro Ile Asp
            20                  25                  30

Val Ser Lys Ala Asp Gly Ile Ile Tyr Pro Gln Gly Arg Thr Tyr Ser
        35                  40                  45

Asn Ile Thr Ile Thr Tyr Gln Gly Leu Phe Pro Tyr Gln Gly Asp His
    50                  55                  60

Gly Asp Met Tyr Val Tyr Ser Ala Gly His Ala Thr Gly Thr Thr Pro
65                  70                  75                  80

Gln Lys Leu Phe Val Ala Asn Tyr Ser Gln Asp Val Lys Gln Phe Ala
                85                  90                  95

Asn Gly Phe Val Val Arg Ile Gly Ala Ala Ala Asn Ser Thr Gly Thr
            100                 105                 110

Val Ile Ile Ser Pro Ser Thr Ser Ala Thr Ile Arg Lys Ile Tyr Pro
        115                 120                 125

Ala Phe Met Leu Gly Ser Ser Val Gly Asn Phe Ser Asp Gly Lys Met
    130                 135                 140

Gly Arg Phe Phe Asn His Thr Leu Val Leu Leu Pro Asp Gly Cys Gly
145                 150                 155                 160

Thr Leu Leu Arg Ala Phe Tyr Cys Ile Leu Glu Pro Arg Ser Gly Asn
                165                 170                 175

His Cys Pro Ala Gly Asn Ser Tyr Thr Ser Phe Ala Thr Tyr His Thr
            180                 185                 190

Pro Ala Thr Asp Cys Ser Asp Gly Asn Tyr Asn Arg Asn Ala Ser Leu
        195                 200                 205

Asn Ser Phe Lys Glu Tyr Phe Asn Leu Arg Asn Cys Thr Phe Met Tyr
    210                 215                 220

Thr Tyr Asn Ile Thr Glu Asp Glu Ile Leu Glu Trp Phe Gly Ile Thr
225                 230                 235                 240

Gln Thr Ala Gln Gly Val His Leu Phe Ser Ser Arg Tyr Val Asp Leu
                245                 250                 255

Tyr Gly Gly Asn Met Phe Gln Phe Ala Thr Leu Pro Val Tyr Asp Thr
            260                 265                 270

Ile Lys Tyr Tyr Ser Ile Ile Pro His Ser Ile Arg Ser Ile Gln Ser
        275                 280                 285

Asp Arg Lys Ala Trp Ala Ala Phe Tyr Val Tyr Lys Leu Gln Pro Leu
    290                 295                 300

Thr Phe Leu Leu Asp Phe Ser Val Asp Gly Tyr Ile Arg Arg Ala Ile
305                 310                 315                 320

Asp Cys Gly Phe Asn Asp Leu Ser Gln Leu His Cys Ser Tyr Glu Ser
                325                 330                 335

Phe Asp Val Glu Ser Gly Val Tyr Ser Val Ser Ser Phe Glu Ala Lys
            340                 345                 350

Pro Ser Gly Ser Val Val Glu Gln Ala Glu Gly Val Glu Cys Asp Phe
        355                 360                 365

Ser Pro Leu Leu Ser Gly Thr Pro Pro Gln Val Tyr Asn Phe Lys Arg

370                 375                 380

Leu Val Phe Thr Asn Cys Asn Tyr Asn Leu Thr Lys Leu Leu Ser Leu
385                 390                 395                 400

Phe Ser Val Asn Asp Phe Thr Cys Ser Gln Ile Ser Pro Ala Ala Ile
                405                 410                 415

Ala Ser Asn Cys Tyr Ser Ser Leu Ile Leu Asp Tyr Phe Ser Tyr Pro
            420                 425                 430

Leu Ser Met Lys Ser Asp Leu Ser Val Ser Ser Ala Gly Pro Ile Ser
        435                 440                 445

Gln Phe Asn Tyr Lys Gln Ser Phe Ser Asn Pro Thr Cys Leu Ile Leu
    450                 455                 460

Ala Thr Val Pro His Asn Leu Thr Thr Ile Thr Lys Pro Leu Lys Tyr
465                 470                 475                 480

Ser Tyr Ile Asn Lys Cys Ser Arg Leu Leu Ser Asp Asp Arg Thr Glu
                485                 490                 495

Val Pro Gln Leu Val Asn Ala Asn Gln Tyr Ser Pro Cys Val Ser Ile
            500                 505                 510

Val Pro Ser Thr Val Trp Glu Asp Gly Asp Tyr Tyr Arg Lys Gln Leu
        515                 520                 525

Ser Pro Leu Glu Gly Gly Gly Trp Leu Val Ala Ser Gly Ser Thr Val
    530                 535                 540

Ala Met Thr Glu Gln Leu Gln Met Gly Phe Gly Ile Thr Val Gln Tyr
545                 550                 555                 560

Gly Thr Asp Thr Asn Ser Val Cys Pro Lys Leu Glu Phe Ala Asn Asp
                565                 570                 575

Thr Lys Ile Ala Ser Gln Leu Gly Asn Cys Val Glu Tyr Ser Leu Tyr
            580                 585                 590

Gly Val Ser Gly Arg Gly Val Phe Gln Asn Cys Thr Ala Val Gly Val
        595                 600                 605

Arg Gln Gln Arg Phe Val Tyr Asp Ala Tyr Gln Asn Leu Val Gly Tyr
    610                 615                 620

Tyr Ser Asp Asp Gly Asn Tyr Tyr Cys Leu Arg Ala Cys Val Ser Val
625                 630                 635                 640

Pro Val Ser Val Ile Tyr Asp Lys Glu Thr Lys Thr His Ala Thr Leu
                645                 650                 655

Phe Gly Ser Val Ala Cys Glu His Ile Ser Ser Thr Met Ser Gln Tyr
            660                 665                 670

Ser Arg Ser Thr Arg Ser Met Leu Lys Arg Arg Asp Ser Thr Tyr Gly
        675                 680                 685

Pro Leu Gln Thr Pro Val Gly Cys Val Leu Gly Leu Val Asn Ser Ser
    690                 695                 700

Leu Phe Val Glu Asp Cys Lys Leu Pro Leu Gly Gln Ser Leu Cys Ala
705                 710                 715                 720

Leu Pro Asp Thr Pro Ser Thr Leu Thr Pro Arg Ser Val Arg
                725                 730

<210>  7
<211>  521
<212>  PRT
<213>  Artificial Sequence

<220>
<223>  S1 [HCoV-229E]

<400>  7

Cys Gln Thr Thr Asn Gly Leu Asn Thr Ser Tyr Ser Val Cys Asn Gly
1               5                   10                  15

Cys Val Gly Tyr Ser Glu Asn Val Phe Ala Val Glu Ser Gly Gly Tyr
            20                  25                  30

Ile Pro Ser Asp Phe Ala Phe Asn Asn Trp Phe Leu Leu Thr Asn Thr
        35                  40                  45

Ser Ser Val Val Asp Gly Val Val Arg Ser Phe Gln Pro Leu Leu Leu
    50                  55                  60

Asn Cys Leu Trp Ser Val Ser Gly Leu Arg Phe Thr Thr Gly Phe Val
65                  70                  75                  80

Tyr Phe Asn Gly Thr Gly Arg Gly Asp Cys Lys Gly Phe Ser Ser Asp
                85                  90                  95

Val Leu Ser Asp Val Ile Arg Tyr Asn Leu Asn Phe Glu Glu Asn Leu

            100                 105                 110

Arg Arg Gly Thr Ile Leu Phe Lys Thr Ser Tyr Gly Val Val Val Phe
        115                 120                 125

Tyr Cys Thr Asn Asn Thr Leu Val Ser Gly Asp Ala His Ile Pro Phe
    130                 135                 140

Gly Thr Val Leu Gly Asn Phe Tyr Cys Phe Val Asn Thr Thr Ile Gly
145                 150                 155                 160

Asn Glu Thr Thr Ser Ala Phe Val Gly Ala Leu Pro Lys Thr Val Arg
                165                 170                 175

Glu Phe Val Ile Ser Arg Thr Gly His Phe Tyr Ile Asn Gly Tyr Arg
            180                 185                 190

Tyr Phe Thr Leu Gly Asn Val Glu Ala Val Asn Phe Asn Val Thr Thr
        195                 200                 205

Ala Glu Thr Thr Asp Phe Cys Thr Val Ala Leu Ala Ser Tyr Ala Asp
    210                 215                 220

Val Leu Val Asn Val Ser Gln Thr Ser Ile Ala Asn Ile Ile Tyr Cys
225                 230                 235                 240

Asn Ser Val Ile Asn Arg Leu Arg Cys Asp Gln Leu Ser Phe Asp Val
                245                 250                 255

Pro Asp Gly Phe Tyr Ser Thr Ser Pro Ile Gln Ser Val Glu Leu Pro
            260                 265                 270

Val Ser Ile Val Ser Leu Pro Val Tyr His Lys His Thr Phe Ile Val
        275                 280                 285

Leu Tyr Val Asp Phe Lys Pro Gln Ser Gly Gly Gly Lys Cys Phe Asn
    290                 295                 300

Cys Tyr Pro Ala Gly Val Asn Ile Thr Leu Ala Asn Phe Asn Glu Thr
305                 310                 315                 320

Lys Gly Pro Leu Cys Val Asp Thr Ser His Phe Thr Thr Lys Tyr Val
                325                 330                 335

Ala Val Tyr Ala Asn Val Gly Arg Trp Ser Ala Ser Ile Asn Thr Gly
            340                 345                 350

Asn Cys Pro Phe Ser Phe Gly Lys Val Asn Asn Phe Val Lys Phe Gly
        355                 360                 365

Ser Val Cys Phe Ser Leu Lys Asp Ile Pro Gly Gly Cys Ala Met Pro
    370                 375                 380

Ile Val Ala Asn Trp Ala Tyr Ser Lys Tyr Tyr Thr Ile Gly Ser Leu
385                 390                 395                 400

Tyr Val Ser Trp Ser Asp Gly Asp Gly Ile Thr Gly Val Pro Gln Pro
                405                 410                 415

Val Glu Gly Val Ser Ser Phe Met Asn Val Thr Leu Asp Lys Cys Thr
            420                 425                 430

Lys Tyr Asn Ile Tyr Asp Val Ser Gly Val Gly Val Ile Arg Val Ser
        435                 440                 445

Asn Asp Thr Phe Leu Asn Gly Ile Thr Tyr Thr Ser Thr Ser Gly Asn
    450                 455                 460

Leu Leu Gly Phe Lys Asp Val Thr Lys Gly Thr Ile Tyr Ser Ile Thr
465                 470                 475                 480

Pro Cys Asn Pro Pro Asp Gln Leu Val Val Tyr Gln Gln Ala Val Val
                485                 490                 495

Gly Ala Met Leu Ser Glu Asn Phe Thr Ser Tyr Gly Phe Ser Asn Val
            500                 505                 510

Val Glu Leu Pro Lys Phe Phe Tyr Ala
        515                 520

<210>  8
<211>  745
<212>  PRT
<213>  Artificial Sequence

<220>
<223>  S1 [HCoV-OC43]

<400>  8

Ala Val Ile Gly Asp Leu Lys Cys Thr Ser Asp Asn Ile Asn Asp Lys
1               5                   10                  15

Asp Thr Gly Pro Pro Pro Ile Ser Thr Asp Thr Val Asp Val Thr Asn
            20                  25                  30

Gly Leu Gly Thr Tyr Tyr Val Leu Asp Arg Val Tyr Leu Asn Thr Thr

35                  40                  45

Leu Phe Leu Asn Gly Tyr Tyr Pro Thr Ser Gly Ser Thr Tyr Arg Asn
    50                  55                  60

Met Ala Leu Lys Gly Ser Val Leu Leu Ser Arg Leu Trp Phe Lys Pro
65                  70                  75                  80

Pro Phe Leu Ser Asp Phe Ile Asn Gly Ile Phe Ala Lys Val Lys Asn
                85                  90                  95

Thr Lys Val Ile Lys Asp Arg Val Met Tyr Ser Glu Phe Pro Ala Ile
            100                 105                 110

Thr Ile Gly Ser Thr Phe Val Asn Thr Ser Tyr Ser Val Val Val Gln
        115                 120                 125

Pro Arg Thr Ile Asn Ser Thr Gln Asp Gly Asp Asn Lys Leu Gln Gly
    130                 135                 140

Leu Leu Glu Val Ser Val Cys Gln Tyr Asn Met Cys Glu Tyr Pro Gln
145                 150                 155                 160

Thr Ile Cys His Pro Asn Leu Gly Asn His Arg Lys Glu Leu Trp His
                165                 170                 175

Leu Asp Thr Gly Val Val Ser Cys Leu Tyr Lys Arg Asn Phe Thr Tyr
            180                 185                 190

Asp Val Asn Ala Asp Tyr Leu Tyr Phe His Phe Tyr Gln Glu Gly Gly
        195                 200                 205

Thr Phe Tyr Ala Tyr Phe Thr Asp Thr Gly Val Val Thr Lys Phe Leu
    210                 215                 220

Phe Asn Val Tyr Leu Gly Met Ala Leu Ser His Tyr Tyr Val Met Pro
225                 230                 235                 240

Leu Thr Cys Asn Ser Lys Leu Thr Leu Glu Tyr Trp Val Thr Pro Leu
                245                 250                 255

Thr Ser Arg Gln Tyr Leu Leu Ala Phe Asn Gln Asp Gly Ile Ile Phe
            260                 265                 270

Asn Ala Glu Asp Cys Met Ser Asp Phe Met Ser Glu Ile Lys Cys Lys
        275                 280                 285

Thr Gln Ser Ile Ala Pro Pro Thr Gly Val Tyr Glu Leu Asn Gly Tyr
    290                 295                 300

Thr Val Gln Pro Ile Ala Asp Val Tyr Arg Arg Lys Pro Asn Leu Pro
305                 310                 315                 320

Asn Cys Asn Ile Glu Ala Trp Leu Asn Asp Lys Ser Val Pro Ser Pro
                325                 330                 335

Leu Asn Trp Glu Arg Lys Thr Phe Ser Asn Cys Asn Phe Asn Met Ser
            340                 345                 350

Ser Leu Met Ser Phe Ile Gln Ala Asp Ser Phe Thr Cys Asn Asn Ile
        355                 360                 365

Asp Ala Ala Lys Ile Tyr Gly Met Cys Phe Ser Ser Ile Thr Ile Asp
    370                 375                 380

Lys Phe Ala Ile Pro Asn Gly Arg Lys Val Asp Leu Gln Leu Gly Asn
385                 390                 395                 400

Leu Gly Tyr Leu Gln Ser Phe Asn Tyr Arg Ile Asp Thr Thr Ala Thr
                405                 410                 415

Ser Cys Gln Leu Tyr Tyr Asn Leu Pro Ala Ala Asn Val Ser Val Ser
            420                 425                 430

Arg Phe Asn Pro Ser Thr Trp Asn Lys Arg Phe Gly Phe Ile Glu Asp
        435                 440                 445

Ser Val Phe Lys Pro Arg Pro Ala Gly Val Leu Thr Asn His Asp Val
    450                 455                 460

Val Tyr Ala Gln His Cys Phe Lys Ala Pro Lys Asn Phe Cys Pro Cys
465                 470                 475                 480

Lys Leu Asn Gly Ser Cys Val Gly Ser Gly Pro Gly Lys Asn Asn Gly
                485                 490                 495

Ile Gly Thr Cys Pro Ala Gly Thr Asn Tyr Leu Thr Cys Asp Asn Leu
            500                 505                 510

Cys Thr Pro Asp Pro Ile Thr Phe Thr Gly Thr Tyr Lys Cys Pro Gln
        515                 520                 525

Thr Lys Ser Leu Val Gly Ile Gly Glu His Cys Ser Gly Leu Ala Val
    530                 535                 540

Lys Ser Asp Tyr Cys Gly Gly Asn Ser Cys Thr Cys Arg Pro Gln Ala
545                 550                 555                 560

Phe Leu Gly Trp Ser Ala Asp Ser Cys Leu Gln Gly Asp Lys Cys Asn
                565                 570                 575

Ile Phe Ala Asn Phe Ile Leu His Asp Val Asn Ser Gly Leu Thr Cys
            580                 585                 590

Ser Thr Asp Leu Gln Lys Ala Asn Thr Asp Ile Ile Leu Gly Val Cys
        595                 600                 605

Val Asn Tyr Asp Leu Tyr Gly Ile Leu Gly Gln Gly Ile Phe Val Glu
    610                 615                 620

Val Asn Ala Thr Tyr Tyr Asn Ser Trp Gln Asn Leu Leu Tyr Asp Ser
625                 630                 635                 640

Asn Gly Asn Leu Tyr Gly Phe Arg Asp Tyr Ile Ile Asn Arg Thr Phe
                645                 650                 655

Met Ile Arg Ser Cys Tyr Ser Gly Arg Val Ser Ala Ala Phe His Ala
            660                 665                 670

Asn Ser Ser Glu Pro Ala Leu Leu Phe Arg Asn Ile Lys Cys Asn Tyr
        675                 680                 685

Val Phe Asn Asn Ser Leu Thr Arg Gln Leu Gln Pro Ile Asn Tyr Phe
    690                 695                 700

Asp Ser Tyr Leu Gly Cys Val Val Asn Ala Tyr Asn Ser Thr Ala Ile
705                 710                 715                 720

Ser Val Gln Thr Cys Asp Leu Thr Val Gly Ser Gly Tyr Cys Val Asp
                725                 730                 735

Tyr Ser Lys Asn Arg Arg Ser Arg Gly
            740                 745

<210>  9
<211>  744
<212>  PRT
<213>  Artificial Sequence

<220>
<223>  S1 [HCoV-HKU1]

<400>  9

Ala Val Ile Gly Asp Phe Asn Cys Thr Asn Ser Phe Ile Asn Asp Tyr
1               5                   10                  15

Asn Lys Thr Ile Pro Arg Ile Ser Glu Asp Val Val Asp Val Ser Leu
            20                  25                  30

Gly Leu Gly Thr Tyr Tyr Val Leu Asn Arg Val Tyr Leu Asn Thr Thr
        35                  40                  45

Leu Leu Phe Thr Gly Tyr Phe Pro Lys Ser Gly Ala Asn Phe Arg Asp
    50                  55                  60

Leu Ala Leu Lys Gly Ser Ile Tyr Leu Ser Thr Leu Trp Tyr Lys Pro
65                  70                  75                  80

Pro Phe Leu Ser Asp Phe Asn Asn Gly Ile Phe Ser Lys Val Lys Asn
                85                  90                  95

Thr Lys Leu Tyr Val Asn Asn Thr Leu Tyr Ser Glu Phe Ser Thr Ile
            100                 105                 110

Val Ile Gly Ser Val Phe Val Asn Thr Ser Tyr Thr Ile Val Val Gln
        115                 120                 125

Pro His Asn Gly Ile Leu Glu Ile Thr Ala Cys Gln Tyr Thr Met Cys
    130                 135                 140

Glu Tyr Pro His Thr Val Cys Lys Ser Lys Gly Ser Ile Arg Asn Glu
145                 150                 155                 160

Ser Trp His Ile Asp Ser Ser Glu Pro Leu Cys Leu Phe Lys Lys Asn
                165                 170                 175

Phe Thr Tyr Asn Val Ser Ala Asp Trp Leu Tyr Phe His Phe Tyr Gln
            180                 185                 190

Glu Arg Gly Val Phe Tyr Ala Tyr Tyr Ala Asp Val Gly Met Pro Thr
        195                 200                 205

Thr Phe Leu Phe Ser Leu Tyr Leu Gly Thr Ile Leu Ser His Tyr Tyr
    210                 215                 220

Val Met Pro Leu Thr Cys Asn Ala Ile Ser Ser Asn Thr Asp Asn Glu
225                 230                 235                 240

Thr Leu Glu Tyr Trp Val Thr Pro Leu Ser Arg Arg Gln Tyr Leu Leu
                245                 250                 255

Asn Phe Asp Glu His Gly Val Ile Thr Asn Ala Val Asp Cys Ser Ser
            260                 265                 270

Ser Phe Leu Ser Glu Ile Gln Cys Lys Thr Gln Ser Phe Ala Pro Asn
        275                 280                 285

Thr Gly Val Tyr Asp Leu Ser Gly Phe Thr Val Lys Pro Val Ala Thr
    290                 295                 300

Val Tyr Arg Arg Ile Pro Asn Leu Pro Asp Cys Asp Ile Asp Asn Trp
305                 310                 315                 320

Leu Asn Asn Val Ser Val Pro Ser Pro Leu Asn Trp Glu Arg Arg Ile
                325                 330                 335

Phe Ser Asn Cys Asn Phe Asn Leu Ser Thr Leu Leu Arg Leu Val His
            340                 345                 350

Val Asp Ser Phe Ser Cys Asn Asn Leu Asp Lys Ser Lys Ile Phe Gly
        355                 360                 365

Ser Cys Phe Asn Ser Ile Thr Val Asp Lys Phe Ala Ile Pro Asn Arg
    370                 375                 380

Arg Arg Asp Asp Leu Gln Leu Gly Ser Ser Gly Phe Leu Gln Ser Ser
385                 390                 395                 400

Asn Tyr Lys Ile Asp Ile Ser Ser Ser Ser Cys Gln Leu Tyr Tyr Ser
                405                 410                 415

Leu Pro Leu Val Asn Val Thr Ile Asn Asn Phe Asn Pro Ser Ser Trp
            420                 425                 430

Asn Arg Arg Tyr Gly Phe Gly Ser Phe Asn Leu Ser Ser Tyr Asp Val
        435                 440                 445

Val Tyr Ser Asp His Cys Phe Ser Val Asn Ser Asp Phe Cys Pro Cys
    450                 455                 460

Ala Asp Pro Ser Val Val Asn Ser Cys Ala Lys Ser Lys Pro Pro Ser
465                 470                 475                 480

Ala Ile Cys Pro Ala Gly Thr Lys Tyr Arg His Cys Asp Leu Asp Thr
                485                 490                 495

Thr Leu Tyr Val Lys Asn Trp Cys Arg Cys Ser Cys Leu Pro Asp Pro
            500                 505                 510

Ile Ser Thr Tyr Ser Pro Asn Thr Cys Pro Gln Lys Lys Val Val Val
        515                 520                 525

Gly Ile Gly Glu His Cys Pro Gly Leu Gly Ile Asn Glu Glu Lys Cys
    530                 535                 540

Gly Thr Gln Leu Asn His Ser Ser Cys Phe Cys Ser Pro Asp Ala Phe
545                 550                 555                 560

Leu Gly Trp Ser Phe Asp Ser Cys Ile Ser Asn Asn Arg Cys Asn Ile
                565                 570                 575

Phe Ser Asn Phe Ile Phe Asn Gly Ile Asn Ser Gly Thr Thr Cys Ser
            580                 585                 590

Asn Asp Leu Leu Tyr Ser Asn Thr Glu Ile Ser Thr Gly Val Cys Val
        595                 600                 605

Asn Tyr Asp Leu Tyr Gly Ile Thr Gly Gln Gly Ile Phe Lys Glu Val
    610                 615                 620

Ser Ala Ala Tyr Tyr Asn Asn Trp Gln Asn Leu Leu Tyr Asp Ser Asn
625                 630                 635                 640

Gly Asn Ile Ile Gly Phe Lys Asp Phe Leu Thr Asn Lys Thr Tyr Thr
                645                 650                 655

Ile Leu Pro Cys Tyr Ser Gly Arg Val Ser Ala Ala Phe Tyr Gln Asn
            660                 665                 670

Ser Ser Ser Pro Ala Leu Leu Tyr Arg Asn Leu Lys Cys Ser Tyr Val
        675                 680                 685

Leu Asn Asn Ile Ser Phe Ile Ser Gln Pro Phe Tyr Phe Asp Ser Tyr
    690                 695                 700

Leu Gly Cys Val Leu Asn Ala Val Asn Leu Thr Ser Tyr Ser Val Ser
705                 710                 715                 720

Ser Cys Asp Leu Arg Met Gly Ser Gly Phe Cys Ile Asp Tyr Ala Leu
                725                 730                 735

Pro Ser Ser Arg Arg Lys Arg Arg
            740

<210>  10

<211>  702
<212>  PRT
<213>  Artificial Sequence

<220>
<223>  S1 [HCoV-NL63]

<400>  10

Phe Phe Thr Cys Asn Ser Asn Ala Asn Leu Ser Met Leu Gln Leu Gly
1               5                   10                  15

Val Pro Asp Asn Ser Ser Thr Ile Val Thr Gly Leu Leu Pro Thr His
            20                  25                  30

Trp Phe Cys Ala Asn Gln Ser Thr Ser Val Tyr Ser Ala Asn Gly Phe
        35                  40                  45

Phe Tyr Ile Asp Val Gly Asn His Arg Ser Ala Phe Ala Leu His Thr
    50                  55                  60

Gly Tyr Tyr Asp Ala Asn Gln Tyr Tyr Ile Tyr Val Thr Asn Glu Ile
65                  70                  75                  80

Gly Leu Asn Ala Ser Val Thr Leu Lys Ile Cys Lys Phe Ser Arg Asn
                85                  90                  95

Thr Thr Phe Asp Phe Leu Ser Asn Ala Ser Ser Ser Phe Asp Cys Ile
            100                 105                 110

Val Asn Leu Leu Phe Thr Glu Gln Leu Gly Ala Pro Leu Gly Ile Thr
        115                 120                 125

Ile Ser Gly Glu Thr Val Arg Leu His Leu Tyr Asn Val Thr Arg Thr
    130                 135                 140

Phe Tyr Val Pro Ala Ala Tyr Lys Leu Thr Lys Leu Ser Val Lys Cys
145                 150                 155                 160

Tyr Phe Asn Tyr Ser Cys Val Phe Ser Val Val Asn Ala Thr Val Thr
                165                 170                 175

Val Asn Val Thr Thr His Asn Gly Arg Val Val Asn Tyr Thr Val Cys
            180                 185                 190

Asp Asp Cys Asn Gly Tyr Thr Asp Asn Ile Phe Ser Val Gln Gln Asp
        195                 200                 205

Gly Arg Ile Pro Asn Gly Phe Pro Phe Asn Asn Trp Phe Leu Leu Thr
    210                 215                 220

Asn Gly Ser Thr Leu Val Asp Gly Val Ser Arg Leu Tyr Gln Pro Leu
225                 230                 235                 240

Arg Leu Thr Cys Leu Trp Pro Val Pro Gly Leu Lys Ser Ser Thr Gly
                245                 250                 255

Phe Val Tyr Phe Asn Ala Thr Gly Ser Asp Val Asn Cys Asn Gly Tyr
            260                 265                 270

Gln His Asn Ser Val Val Asp Val Met Arg Tyr Asn Leu Asn Phe Ser
        275                 280                 285

Ala Asn Ser Leu Asp Asn Leu Lys Ser Gly Val Ile Val Phe Lys Thr
    290                 295                 300

Leu Gln Tyr Asp Val Leu Phe Tyr Cys Ser Asn Ser Ser Ser Gly Val
305                 310                 315                 320

Leu Asp Thr Thr Ile Pro Phe Gly Pro Ser Ser Gln Pro Tyr Tyr Cys
                325                 330                 335

Phe Ile Asn Ser Thr Ile Asn Thr Thr His Val Ser Thr Phe Val Gly
            340                 345                 350

Ile Leu Pro Pro Thr Val Arg Glu Ile Val Val Ala Arg Thr Gly Gln
        355                 360                 365

Phe Tyr Ile Asn Gly Phe Lys Tyr Phe Asp Leu Gly Phe Ile Glu Ala
    370                 375                 380

Val Asn Phe Asn Val Thr Thr Ala Ser Ala Thr Asp Phe Trp Thr Val
385                 390                 395                 400

Ala Phe Ala Thr Phe Val Asp Val Leu Val Asn Val Ser Ala Thr Asn
                405                 410                 415

Ile Gln Asn Leu Leu Tyr Cys Asp Ser Pro Phe Glu Lys Leu Gln Cys
            420                 425                 430

Glu His Leu Gln Phe Gly Leu Gln Asp Gly Phe Tyr Ser Ala Asn Phe
        435                 440                 445

Leu Asp Asp Asn Val Leu Pro Glu Thr Tyr Val Ala Leu Pro Ile Tyr
    450                 455                 460

Tyr Gln His Thr Asp Ile Asn Phe Thr Ala Thr Ala Ser Phe Gly Gly

465                 470                 475                 480

Ser Cys Tyr Val Cys Lys Pro His Gln Val Asn Ile Ser Leu Asn Gly
                485                 490                 495

Asn Thr Ser Val Cys Val Arg Thr Ser His Phe Ser Ile Arg Tyr Ile
            500                 505                 510

Tyr Asn Arg Val Lys Ser Gly Ser Pro Gly Asp Ser Ser Trp His Ile
        515                 520                 525

Tyr Leu Lys Ser Gly Thr Cys Pro Phe Ser Phe Ser Lys Leu Asn Asn
    530                 535                 540

Phe Gln Lys Phe Lys Thr Ile Cys Phe Ser Thr Val Glu Val Pro Gly
545                 550                 555                 560

Ser Cys Asn Phe Pro Leu Glu Ala Thr Trp His Tyr Thr Ser Tyr Thr
                565                 570                 575

Ile Val Gly Ala Leu Tyr Val Thr Trp Ser Glu Gly Asn Ser Ile Thr
            580                 585                 590

Gly Val Pro Tyr Pro Val Ser Gly Ile Arg Glu Phe Ser Asn Leu Val
        595                 600                 605

Leu Asn Asn Cys Thr Lys Tyr Asn Ile Tyr Asp Tyr Val Gly Thr Gly
    610                 615                 620

Ile Ile Arg Ser Ser Asn Gln Ser Leu Ala Gly Gly Ile Thr Tyr Val
625                 630                 635                 640

Ser Asn Ser Gly Asn Leu Leu Gly Phe Lys Asn Val Ser Thr Gly Asn
                645                 650                 655

Ile Phe Ile Val Thr Pro Cys Asn Gln Pro Asp Gln Val Ala Val Tyr
            660                 665                 670

Gln Gln Ser Ile Ile Gly Ala Met Thr Ala Val Asn Glu Ser Arg Tyr
        675                 680                 685

Gly Leu Gln Asn Leu Leu Gln Leu Pro Asn Phe Tyr Tyr Val
    690                 695                 700

<210>  11
<211>  657
<212>  PRT
<213>  Artificial Sequence

<220>
<223>  S1 [SARS_CoV]

<400>  11

Ser Gly Ser Asp Leu Asp Arg Cys Thr Thr Phe Asp Asp Val Gln Ala
1               5                   10                  15

Pro Asn Tyr Thr Gln His Thr Ser Ser Met Arg Gly Val Tyr Tyr Pro
            20                  25                  30

Asp Glu Ile Phe Arg Ser Asp Thr Leu Tyr Leu Thr Gln Asp Leu Phe
        35                  40                  45

Leu Pro Phe Tyr Ser Asn Val Thr Gly Phe His Thr Ile Asn His Thr
    50                  55                  60

Phe Gly Asn Pro Val Ile Pro Phe Lys Asp Gly Ile Tyr Phe Ala Ala
65                  70                  75                  80

Thr Glu Lys Ser Asn Val Val Arg Gly Trp Val Phe Gly Ser Thr Met
                85                  90                  95

Asn Asn Lys Ser Gln Ser Val Ile Ile Ile Asn Asn Ser Thr Asn Val
            100                 105                 110

Val Ile Arg Ala Cys Asn Phe Glu Leu Cys Asp Asn Pro Phe Phe Ala
        115                 120                 125

Val Ser Lys Pro Met Gly Thr Gln Thr His Thr Met Ile Phe Asp Asn
    130                 135                 140

Ala Phe Asn Cys Thr Phe Glu Tyr Ile Ser Asp Ala Phe Ser Leu Asp
145                 150                 155                 160

Val Ser Glu Lys Ser Gly Asn Phe Lys His Leu Arg Glu Phe Val Phe
                165                 170                 175

Lys Asn Lys Asp Gly Phe Leu Tyr Val Tyr Lys Gly Tyr Gln Pro Ile
            180                 185                 190

Asp Val Val Arg Asp Leu Pro Ser Gly Phe Asn Thr Leu Lys Pro Ile
        195                 200                 205

Phe Lys Leu Pro Leu Gly Ile Asn Ile Thr Asn Phe Arg Ala Ile Leu
    210                 215                 220

Thr Ala Phe Ser Pro Ala Gln Asp Ile Trp Gly Thr Ser Ala Ala Ala

225                 230                 235                 240

Tyr Phe Val Gly Tyr Leu Lys Pro Thr Thr Phe Met Leu Lys Tyr Asp
                245                 250                 255

Glu Asn Gly Thr Ile Thr Asp Ala Val Asp Cys Ser Gln Asn Pro Leu
            260                 265                 270

Ala Glu Leu Lys Cys Ser Val Lys Ser Phe Glu Ile Asp Lys Gly Ile
        275                 280                 285

Tyr Gln Thr Ser Asn Phe Arg Val Val Pro Ser Gly Asp Val Val Arg
    290                 295                 300

Phe Pro Asn Ile Thr Asn Leu Cys Pro Phe Gly Glu Val Phe Asn Ala
305                 310                 315                 320

Thr Lys Phe Pro Ser Val Tyr Ala Trp Glu Arg Lys Lys Ile Ser Asn
                325                 330                 335

Cys Val Ala Asp Tyr Ser Val Leu Tyr Asn Ser Thr Phe Phe Ser Thr
            340                 345                 350

Phe Lys Cys Tyr Gly Val Ser Ala Thr Lys Leu Asn Asp Leu Cys Phe
        355                 360                 365

Ser Asn Val Tyr Ala Asp Ser Phe Val Val Lys Gly Asp Asp Val Arg
    370                 375                 380

Gln Ile Ala Pro Gly Gln Thr Gly Val Ile Ala Asp Tyr Asn Tyr Lys
385                 390                 395                 400

Leu Pro Asp Asp Phe Met Gly Cys Val Leu Ala Trp Asn Thr Arg Asn
                405                 410                 415

Ile Asp Ala Thr Ser Thr Gly Asn Tyr Asn Tyr Lys Tyr Arg Tyr Leu
            420                 425                 430

Arg His Gly Lys Leu Arg Pro Phe Glu Arg Asp Ile Ser Asn Val Pro
        435                 440                 445

Phe Ser Pro Asp Gly Lys Pro Cys Thr Pro Pro Ala Leu Asn Cys Tyr
    450                 455                 460

Trp Pro Leu Asn Asp Tyr Gly Phe Tyr Thr Thr Thr Gly Ile Gly Tyr
465                 470                 475                 480

Gln Pro Tyr Arg Val Val Val Leu Ser Phe Glu Leu Leu Asn Ala Pro
                485                 490                 495

Ala Thr Val Cys Gly Pro Lys Leu Ser Thr Asp Leu Ile Lys Asn Gln
            500                 505                 510

Cys Val Asn Phe Asn Phe Asn Gly Leu Thr Gly Thr Gly Val Leu Thr
        515                 520                 525

Pro Ser Ser Lys Arg Phe Gln Pro Phe Gln Gln Phe Gly Arg Asp Val
    530                 535                 540

Ser Asp Phe Thr Asp Ser Val Arg Asp Pro Lys Thr Ser Glu Ile Leu
545                 550                 555                 560

Asp Ile Ser Pro Cys Ser Phe Gly Gly Val Ser Val Ile Thr Pro Gly
                565                 570                 575

Thr Asn Ala Ser Ser Glu Val Ala Val Leu Tyr Gln Asp Val Asn Cys
            580                 585                 590

Thr Asp Val Ser Thr Ala Ile His Ala Asp Gln Leu Thr Pro Ala Trp
        595                 600                 605

Arg Ile Tyr Ser Thr Gly Asn Asn Val Phe Gln Thr Gln Ala Gly Cys
    610                 615                 620

Leu Ile Gly Ala Glu His Val Asp Thr Ser Tyr Glu Cys Asp Ile Pro
625                 630                 635                 640

Ile Gly Ala Gly Ile Cys Ala Ser Tyr His Thr Val Ser Leu Leu Arg
                645                 650                 655

Leu

<210>  12
<211>  649
<212>  PRT
<213>  Artificial Sequence

<220>
<223>  fragment of SEQ ID NO1

<400>  12

Asn Ser Phe Thr Arg Gly Val Tyr Tyr Pro Asp Lys Val Phe Arg Ser
1               5                   10                  15

Ser Val Leu His Ser Thr Gln Asp Leu Phe Leu Pro Phe Phe Ser Asn

            20                  25                  30

Val Thr Trp Phe His Ala Ile His Val Ser Gly Thr Asn Gly Thr Lys
        35                  40                  45

Arg Phe Asp Asn Pro Val Leu Pro Phe Asn Asp Gly Val Tyr Phe Ala
    50                  55                  60

Ser Thr Glu Lys Ser Asn Ile Ile Arg Gly Trp Ile Phe Gly Thr Thr
65                  70                  75                  80

Leu Asp Ser Lys Thr Gln Ser Leu Leu Ile Val Asn Asn Ala Thr Asn
                85                  90                  95

Val Val Ile Lys Val Cys Glu Phe Gln Phe Cys Asn Asp Pro Phe Leu
            100                 105                 110

Gly Val Tyr Tyr His Lys Asn Asn Lys Ser Trp Met Glu Ser Glu Phe
        115                 120                 125

Arg Val Tyr Ser Ser Ala Asn Asn Cys Thr Phe Glu Tyr Val Ser Gln
    130                 135                 140

Pro Phe Leu Met Asp Leu Glu Gly Lys Gln Gly Asn Phe Lys Asn Leu
145                 150                 155                 160

Arg Glu Phe Val Phe Lys Asn Ile Asp Gly Tyr Phe Lys Ile Tyr Ser
                165                 170                 175

Lys His Thr Pro Ile Asn Leu Val Arg Asp Leu Pro Gln Gly Phe Ser
            180                 185                 190

Ala Leu Glu Pro Leu Val Asp Leu Pro Ile Gly Ile Asn Ile Thr Arg
        195                 200                 205

Phe Gln Thr Leu Leu Ala Leu His Arg Ser Tyr Leu Thr Pro Gly Asp
    210                 215                 220

Ser Ser Ser Gly Trp Thr Ala Gly Ala Ala Ala Tyr Tyr Val Gly Tyr
225                 230                 235                 240

Leu Gln Pro Arg Thr Phe Leu Leu Lys Tyr Asn Glu Asn Gly Thr Ile
                245                 250                 255

Thr Asp Ala Val Asp Cys Ala Leu Asp Pro Leu Ser Glu Thr Lys Cys
            260                 265                 270

Thr Leu Lys Ser Phe Thr Val Glu Lys Gly Ile Tyr Gln Thr Ser Asn
        275                 280                 285

Phe Arg Val Gln Pro Thr Glu Ser Ile Val Arg Phe Pro Asn Ile Thr
    290                 295                 300

Asn Leu Cys Pro Phe Gly Glu Val Phe Asn Ala Thr Arg Phe Ala Ser
305                 310                 315                 320

Val Tyr Ala Trp Asn Arg Lys Arg Ile Ser Asn Cys Val Ala Asp Tyr
                325                 330                 335

Ser Val Leu Tyr Asn Ser Ala Ser Phe Ser Thr Phe Lys Cys Tyr Gly
            340                 345                 350

Val Ser Pro Thr Lys Leu Asn Asp Leu Cys Phe Thr Asn Val Tyr Ala
        355                 360                 365

Asp Ser Phe Val Ile Arg Gly Asp Glu Val Arg Gln Ile Ala Pro Gly
    370                 375                 380

Gln Thr Gly Lys Ile Ala Asp Tyr Asn Tyr Lys Leu Pro Asp Asp Phe
385                 390                 395                 400

Thr Gly Cys Val Ile Ala Trp Asn Ser Asn Asn Leu Asp Ser Lys Val
                405                 410                 415

Gly Gly Asn Tyr Asn Tyr Leu Tyr Arg Leu Phe Arg Lys Ser Asn Leu
            420                 425                 430

Lys Pro Phe Glu Arg Asp Ile Ser Thr Glu Ile Tyr Gln Ala Gly Ser
        435                 440                 445

Thr Pro Cys Asn Gly Val Glu Gly Phe Asn Cys Tyr Phe Pro Leu Gln
    450                 455                 460

Ser Tyr Gly Phe Gln Pro Thr Asn Gly Val Gly Tyr Gln Pro Tyr Arg
465                 470                 475                 480

Val Val Val Leu Ser Phe Glu Leu Leu His Ala Pro Ala Thr Val Cys
                485                 490                 495

Gly Pro Lys Lys Ser Thr Asn Leu Val Lys Asn Lys Cys Val Asn Phe
            500                 505                 510

Asn Phe Asn Gly Leu Thr Gly Thr Gly Val Leu Thr Glu Ser Asn Lys
        515                 520                 525

Lys Phe Leu Pro Phe Gln Gln Phe Gly Arg Asp Ile Ala Asp Thr Thr
    530                 535                 540

Asp Ala Val Arg Asp Pro Gln Thr Leu Glu Ile Leu Asp Ile Thr Pro
545                 550                 555                 560

Cys Ser Phe Gly Gly Val Ser Val Ile Thr Pro Gly Thr Asn Thr Ser
                565                 570                 575

Asn Gln Val Ala Val Leu Tyr Gln Asp Val Asn Cys Thr Glu Val Pro
            580                 585                 590

Val Ala Ile His Ala Asp Gln Leu Thr Pro Thr Trp Arg Val Tyr Ser
        595                 600                 605

Thr Gly Ser Asn Val Phe Gln Thr Arg Ala Gly Cys Leu Ile Gly Ala
    610                 615                 620

Glu His Val Asn Asn Ser Tyr Glu Cys Asp Ile Pro Ile Gly Ala Gly
625                 630                 635                 640

Ile Cys Ala Ser Tyr Gln Thr Gln Thr
                645